(19) Europäisches Patentamt / European Patent Office / Office européen des brevets

(11) **EP 2 512 219 B1**

(12) **EUROPEAN PATENT SPECIFICATION**

(45) Date of publication and mention
of the grant of the patent:
**03.10.2018 Bulletin 2018/40**

(51) Int Cl.:
*A01H 5/00* (2018.01)  *A01H 1/00* (2006.01)
*A01P 7/00* (2006.01)  *A01H 5/10* (2018.01)
*C12N 15/82* (2006.01)  *A01N 63/02* (2006.01)

(21) Application number: **10838257.3**

(22) Date of filing: **16.12.2010**

(86) International application number:
**PCT/US2010/060810**

(87) International publication number:
**WO 2011/075585 (23.06.2011 Gazette 2011/25)**

(54) **COMBINED USE OF VIP3AB AND CRY1FA FOR MANAGEMENT OF RESISTANT INSECTS**

KOMBINIERTE VERWENDUNG VON VIP3AB UND CRY1FA ZUR BEKÄMPFUNG VON
RESISTENTEN INSEKTEN

UTILISATION COMBINÉE DE VIP3AB ET CRY1FA POUR LA GESTION DES INSECTES
RÉSISTANTS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**ME**

(30) Priority: **16.12.2009 US 284290 P
16.12.2009 US 284252 P
16.12.2009 US 284281 P
16.12.2009 US 284278 P**

(43) Date of publication of application:
**24.10.2012 Bulletin 2012/43**

(73) Proprietor: **Dow AgroSciences, LLC
Indianapolis, IN 46268 (US)**

(72) Inventors:
 • **MEADE, Thomas
 Zionsville
 IN 46077 (US)**
 • **NARVA, Kenneth
 Zionsville
 IN 46077 (US)**
 • **STORER, Nicholas, P.
 Kensington
 MD 20895 (US)**
 • **SHEETS, Joel, J.
 Zionsville
 IN 46077 (US)**
 • **WOOSLEY, Aaron, T.
 Fishers
 IN 46038 (US)**
 • **BURTON, Stephanie, L.
 Indianapolis
 IN 46278 (US)**

(74) Representative: **Zwicker, Jörk et al
ZSP Patentanwälte PartG mbB
Hansastraße 32
80686 München (DE)**

(56) References cited:
 WO-A1-2005/103266   WO-A1-2009/132850
 US-A- 5 723 758     US-A1- 2003 084 606
 US-A1- 2004 133 942  US-A1- 2005 155 103
 US-A1- 2005 216 969  US-A1- 2007 006 340
 US-A1- 2010 235 951

 • BRAVO A ET AL: "How to cope with insect
 resistance to Bt toxins?", TRENDS IN
 BIOTECHNOLOGY, ELSEVIER PUBLICATIONS,
 CAMBRIDGE, GB, vol. 26, no. 10, 1 October 2008
 (2008-10-01), pages 573-579, XP025406825, ISSN:
 0167-7799, DOI: 10.1016/J.TIBTECH.2008.06.005
 [retrieved on 2008-08-14]
 • GUTIERREZ ET AL.: 'Physiologically based
 demographics of Bt cotton-pest interactions I.
 Pink bollworm resistance, refuge and risk'
 ECOLOGICAL MODELLING vol. 191, no. 3-4, 05
 February 2006, pages 346 - 359, XP005239868

- DATABASE GENBANK [Online] 26 August 1998 'Bacillus thuringiensis Cry1Be1 delta-endotoxin gene, complete cds. http://www.ncbi.nlm.nih.gov/nuccore/AF077326' Database accession no. AF077326.1
- CRICKMORE N ET AL: "Revision of the nomenclature for the Bacillus thuringiensis pesticidal crystal proteins", MICROBIOLOGY AND MOLECULAR BIOLOGY REVIEWS, vol. 62, no. 3, September 1998 (1998-09), pages 807-813, ISSN: 1092-2172

Remarks:
The file contains technical information submitted after the application was filed and not included in this specification

**Description**

Background of the Invention

Humans grow corn for food and energy applications. Humans also grow many other crops, including soybeans and cotton. Insects eat and damage plants and thereby undermine these human efforts. Billions of dollars are spent each year to control insect pests and additional billions are lost to the damage they inflict. Synthetic organic chemical insecticides have been the primary tools used to control insect pests but biological insecticides, such as the insecticidal proteins derived from *Bacillus thuringiensis* (*Bt*), have played an important role in some areas. The ability to produce insect-resistant plants through transformation with *Bt* insecticidal protein genes has revolutionized modern agriculture and heightened the importance and value of insecticidal proteins and their genes.

Several *Bt* proteins have been used to create the insect-resistant transgenic plants that have been successfully registered and commercialized to date. These include Cry1Ab, Cry1Ac, Cry1F and Cry3Bb in corn, Cry1Ac and Cry2Ab in cotton, and Cry3A in potato.

The commercial products expressing these proteins express a single protein except in cases where the combined insecticidal spectrum of 2 proteins is desired (*e.g.,* Cry1Ab and Cry3Bb in corn combined to provide resistance to lepidopteran pests and rootworm, respectively) or where the independent action of the proteins makes them useful as a tool for delaying the development of resistance in susceptible insect populations (*e.g.,* Cry1Ac and Cry2Ab in cotton combined to provide resistance management for tobacco budworm). *See also* US 2009 0313717, which relates to a Cry2 protein plus a Vip3Aa, Cry1F, or Cry1A for control of *Helicoverpa zea* or *armigerain.* WO 2009 132850 relates to Cry1F or Cry1A and Vip3Aa for controlling *Spodoptera frugiperda.* US 2008 0311096 relates in part to Cry1Ab for controlling Cry1F-resistant ECB.

That is, some of the qualities of insect-resistant transgenic plants that have led to rapid and widespread adoption of this technology also give rise to the concern that pest populations will develop resistance to the insecticidal proteins produced by these plants. Several strategies have been suggested for preserving the utility of *Bt*-based insect resistance traits which include deploying proteins at a high dose in combination with a refuge, and alternation with, or co-deployment of, different toxins (McGaughey et al. (1998), "B.t. Resistance Management," Nature Biotechnol. 16:144-146).

The proteins selected for use in an IRM stack need to exert their insecticidal effect independently so that resistance developed to one protein does not confer resistance to the second protein (*i.e.,* there is not cross resistance to the proteins). If, for example, a pest population selected for resistance to "Protein A" is sensitive to "Protein B", one would conclude that there is not cross resistance and that a combination of Protein A and Protein B would be effective in delaying resistance to Protein A alone.

In the absence of resistant insect populations, assessments can be made based on other characteristics presumed to be related to mechanism of action and cross-resistance potential. The utility of receptor-mediated binding in identifying insecticidal proteins likely to not exhibit cross resistance has been suggested (van Mellaert *et al.* 1999). The key predictor of lack of cross resistance inherent in this approach is that the insecticidal proteins do not compete for receptors in a sensitive insect species.

In the event that two *Bt* toxins compete for the same receptor, then if that receptor mutates in that insect so that one of the toxins no longer binds to that receptor and thus is no longer insecticidal against the insect, it might be the case that the insect will also be resistant to the second toxin (which competitively bound to the same receptor). That is, the insect is said to be cross-resistant to both *Bt* toxins. However, if two toxins bind to two different receptors, this could be an indication that the insect would not be simultaneously resistant to those two toxins.

Cry1Fa is useful in controlling many lepidopteran pests species including the European corn borer (ECB; *Ostrinia nubilalis* (Hübner)) and the fall armyworm (FAW; *Spodoptera frugiperda*), and is active against the sugarcane borer (SCB; *Diatraea saccharalis).* The Cry1Fa protein, as produced in corn plants containing event TC1507, is responsible for an industry-leading insect resistance trait for FAW control. Cry1Fa is further deployed in the Herculex®, Smart-Stax™, and WideStrike™ products.

The ability to conduct (competitive or homologous) receptor binding studies using Cry1Fa protein is limited because the most common technique available for labeling proteins for detection in receptor binding assays inactivates the insecticidal activity of the Cry1Fa protein.

Additional *Cry* toxins are listed at the website of the official *B.t.* nomenclature committee (Crickmore *et al.;* lifesci.sussex.ac.uk/home/Neil_Crickmore/Bt/). There are currently nearly 60 main groups of "Cry" toxins (Cry1-Cry59), with additional Cyt toxins and VIP toxins and the like. Many of each numeric group have capital-letter subgroups, and the capital letter subgroups have lower-cased letter sub-subgroups. (Cry1 has A-L, and Cry1A has a-i, for example).

Brief Summary of the Invention

The subject invention relates in part to the surprising discovery that a fall armyworm (*Spodoptera frugiperda;*

FAW) population resistant to the insecticidal activity of the Cry1Fa protein is not resistant to the insecticidal activity of the Vip3Ab protein. The subject pair of toxins provides non-cross-resistant action against FAW.

As one skilled in the art will recognize with the benefit of this disclosure, plants expressing Vip3Ab and Cry1Fa, or insecticidal portions thereof, will be useful in delaying or preventing the development of resistance to either of these insecticidal proteins alone.

The subject invention is also supported by the discovery that Vip3Ab and Cry1Fa do not compete with each other for binding sites in the gut of FAW.

Thus, the subject invention relates in part to the use of a Vip3Ab protein in combination with a Cry1Fa protein. Plants (and acreage planted with such plants) that produce Vip3Ab plus Cry1Fa are included within the scope of the subject invention.

The subject invention also relates in part to triple stacks or "pyramids" of three toxins, or more, with Vip3Ab and Cry1Fa being the base pair. In some preferred pyramid embodiments, the selected toxin(s) have non-cross-resistant action against FAW. Some preferred proteins for these triple-stack pyramid combinations are Cry1Fa plus Vip3Ab plus Cry1C, Cry1D, or Cry1Be. These particular triple stacks would, according to the subject invention, advantageously and surprisingly provide non-cross-resistant action against FAW. This can help to reduce or eliminate the requirement for refuge acreage.

With Cry1Fa being active against both FAW and European cornborer (ECB), and in light of the data presented herein, a quad (four-way) stack could also be selected to provide four proteins, wherein three of the four have non-cross-resistant activity against ECB, and three of the four have non-cross-resistant activity against FAW. This could be obtained by using Cry1Be (active against both ECB and FAW) together with the subject pair of proteins, plus one additional protein that is active against ECB. Such quad stacks, according to the subject invention, are:

Cry1F plus Cry1Be plus Vip3Ab (active against FAW) plus Cry1Ab, Cry2A, Cry1I, or DIG-3 (active against ECB).

BRIEF DESCRIPTION OF THE FIGURES

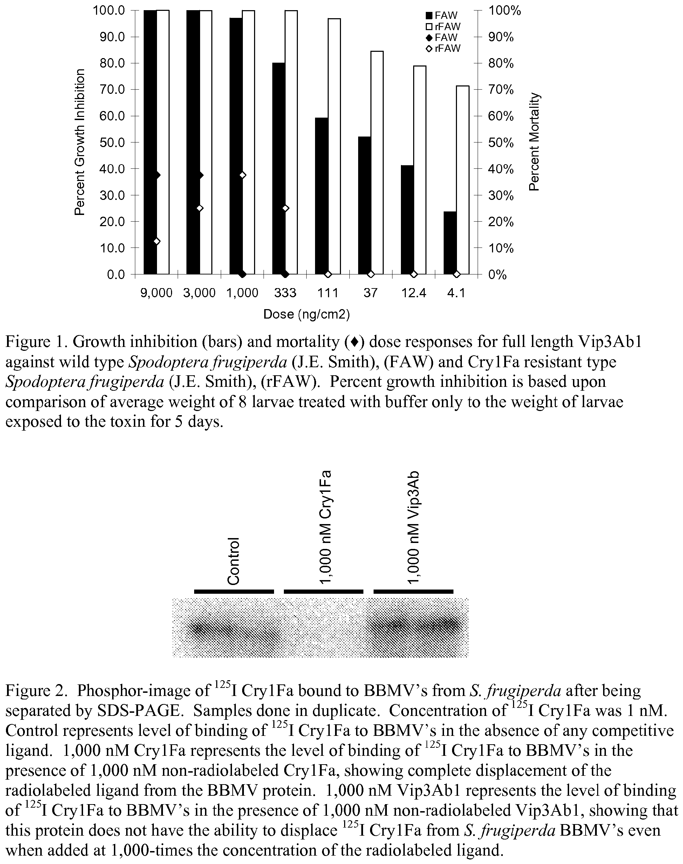

Figure 1. Growth inhibition (bars) and mortality (♦) dose responses for full length Vip3Ab1 against wild type *Spodoptera frugiperda* (J.E. Smith), (FAW) and Cry1Fa resistant type *Spodoptera frugiperda* (J.E. Smith), (rFAW). Percent growth inhibition is based upon comparison of average weight of 8 larvae treated with buffer only to the weight of larvae exposed to the toxin for 5 days.

Figure 2. Phosphor-image of $^{125}$I Cry1Fa bound to BBMV's from *S. frugiperda* after being separated by SDS-PAGE. Samples done in duplicate. Concentration of $^{125}$I Cry1Fa was 1 nM. Control represents level of binding of $^{125}$I Cry1Fa to BBMV's in the absence of any competitive ligand. 1,000 nM Cry1Fa represents the level of binding of $^{125}$I Cry1Fa to BBMV's in the presence of 1,000 nM non-radiolabeled Cry1Fa, showing complete displacement of the radiolabeled ligand from the BBMV protein. 1,000 nM Vip3Ab1 represents the level of binding of $^{125}$I Cry1Fa to BBMV's in the presence of 1,000 nM non-radiolabeled Vip3Ab1, showing that this protein does not have the ability to displace $^{125}$I Cry1Fa from *S. frugiperda* BBMV's even when added at 1,000-times the concentration of the radiolabeled ligand.

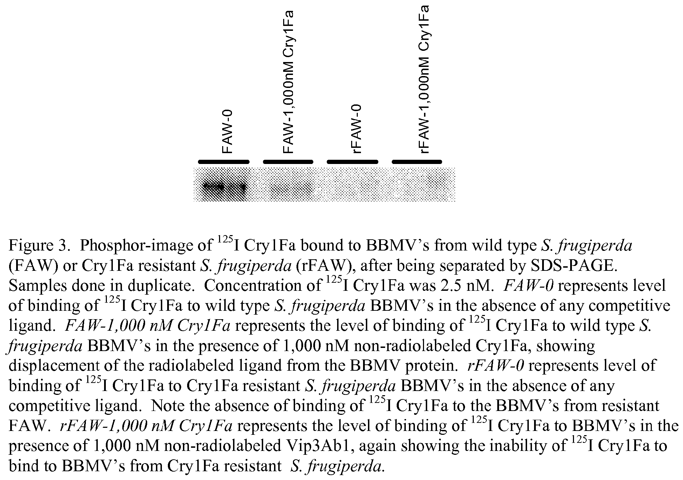

Figure 3. Phosphor-image of $^{125}$I Cry1Fa bound to BBMV's from wild type *S. frugiperda* (FAW) or Cry1Fa resistant *S. frugiperda* (rFAW), after being separated by SDS-PAGE. Samples done in duplicate. Concentration of $^{125}$I Cry1Fa was 2.5 nM. *FAW-0* represents level of binding of $^{125}$I Cry1Fa to wild type *S. frugiperda* BBMV's in the absence of any competitive ligand. *FAW-1,000 nM Cry1Fa* represents the level of binding of $^{125}$I Cry1Fa to wild type *S. frugiperda* BBMV's in the presence of 1,000 nM non-radiolabeled Cry1Fa, showing displacement of the radiolabeled ligand from the BBMV protein. *rFAW-0* represents level of binding of $^{125}$I Cry1Fa to Cry1Fa resistant *S. frugiperda* BBMV's in the absence of any competitive ligand. Note the absence of binding of $^{125}$I Cry1Fa to the BBMV's from resistant FAW. *rFAW-1,000 nM Cry1Fa* represents the level of binding of $^{125}$I Cry1Fa to BBMV's in the presence of 1,000 nM non-radiolabeled Vip3Ab1, again showing the inability of $^{125}$I Cry1Fa to bind to BBMV's from Cry1Fa resistant *S. frugiperda.*

DETAILED DESCRIPTION OF THE INVENTION

As reported herein, a Vip3Ab toxin produced in transgenic corn (and other plants; cotton and soybeans, for example) can be very effective in controlling fall armyworm (FAW; *Spodoptera frugiperda)* that have developed resistance to Cry1Fa activity. Thus, the subject invention relates in part to the surprising discovery that fall armyworm resistant to Cry1Fa are susceptible (*i.e.,* are not cross-resistant) to Vip3Ab. Stated another way, the subject invention also relates in part to the surprising discovery that Vip3Ab toxin is effective at protecting plants (such as maize plants) from damage by Cry1Fa-resistant fall armyworm. For a discussion of this pest, *see e.g.* Tabashnik, PNAS (2008), vol. 105 no. 49, 19029-19030.

The subject invention includes the use of Vip3Ab toxin to protect corn and other economically important plant species (such as soybeans) from damage and yield loss caused by fall armyworm feeding or to fall armyworm populations that have developed resistance to Cry1Fa.

The subject invention thus teaches an IRM stack comprising Vip3Ab to prevent or mitigate the development of resistance by fall armyworm to Cry1Fa.

The present invention provides compositions for controlling lepidopteran pests comprising cells that produce a Cry1Fa core toxin-containing protein and a Vip3Ab core toxin-containing protein.

The invention further comprises a host transformed to produce both a Cry1Fa insecticidal protein and a Vip3Ab insecticidal protein, wherein said host is a plant. The subject polynucleotide(s) are preferably in a genetic construct under control of (operably linked to / comprising) a non-*Bacillus-thuringiensis* promoter(s). The subject polynucleotides can comprise codon usage for enhanced expression in a plant.

It is additionally intended that the disclosure provides a method of controlling lepidopteran pests comprising contacting said pests or the environment of said pests with an effective amount of a composition that contains a Cry1Fa core toxin-containing protein and further contains a Vip3Ab core toxin-containing protein.

A disclosure comprises a maize plant comprising a plant-expressible gene encoding a Vip3Ab core toxin-containing protein and a plant-expressible gene encoding a Cry1Fa core toxin-containing protein, and seed of such a plant.

A further disclosure comprises a maize plant wherein a plant-expressible gene encoding a Vip3Ab core toxin-containing protein and a plant-expressible gene encoding a Cry1Fa core toxin-containing protein have been introgressed into said maize plant, and seed of such a plant.

As described in the Examples, competitive binding studies using radiolabeled Vip3Ab core toxin protein show that the Cry1Fa core toxin protein does not compete for binding in FAW insect tissues to which Vip3Ab binds. These results also indicate that the combination of Cry1Fa and Vip3Ab proteins is an effective means to mitigate the development of resistance in FAW populations to Cry1Fa (and likewise, the development of resistance to Vip3Ab), and would likely increase the level of resistance to this pest in corn plants expressing both proteins. Thus, based in part on the data described herein, it is thought that co-production (stacking) of the Vip3Ab and Cry1Fa proteins can be used to produce a high dose IRM stack for FAW. With Cry1Fa being active againt both FAW and European cornborer (ECB), the subject pair of toxins provides non-competitive action against the FAW.

Other proteins can be added to this pair to expand insect-control spectrum. Another deployment option would be to use Cry1Fa and Vip3Ab proteins in combination with another, third toxin/gene, and to use this triple stack to mitigate the development of resistance in FAW to any of these toxins. Thus, another deployment option of the subject invention would be to use two, three, or more proteins in crop-growing regions where FAW can develop resistant populations.

Accordingly, the subject invention also relates in part to triple stacks or "pyramids" of three (or more) toxins, with Cry1Fa and Vip3Ab toxins being the base pair.

In some preferred pyramid embodiments, the three selected proteins provide non-cross-resistant action against FAW. Some preferred "triple action" pyramid combinations are Cry1Fa plus Vip3Ab plus either Cry1C or Cry1D. *See* USSN 61/284,281 (filed December 16, 2009), which shows that Cry1C is active against Cry1F-resistant FAW, and USSN 61/284,252 (filed December 16, 2009), which shows that Cry1D is active against Cry1F-resistant FAW. These two applications also show that Cry1C does not compete with Cry1F for binding in FAW membrane preparations, and that Cry1D does not compete with Cry1F for binding in FAW membrane preparations. In some embodiments, Cry1Be or Cry1E could be combined with Vip3A and Cry1F as the third anti-FAW protein. For use of Cry1Be with Cry1F, see USSN 61/284,290 (filed December 16, 2009). For use of Cry1E with Cry1F, see USSN 61/284,278 (filed December 16, 2009). These particular triple stacks would, according to the subject invention, advantageously and surprisingly provide three proteins providing non-cross-resistant action against FAW. This can help to reduce or eliminate the requirement for refuge acreage.

In light of the data presented herein, a quad (four-way) stack could also be selected to provide three proteins with non-cross-resistant action against ECB and three proteins with non-cross-resistant action against FAW. This could be obtained by using Cry1Be (active against both ECB and FAW) together with Cry1Fa (active against both ECB and FAW) together with the subject Vip3Ab (active against FAW) and a fourth protein - having ECB-toxicity (*See* USSN 61/284,290, filed December 16, 2009, which relates to combinations of Cry1Fa and Cry1Be.) Examples of quad stacks, according to the subject invention, are:

Cry1F plus Cry1Be plus Vip3 (active against FAW) plus (Cry1Ab, Cry2A, Cry1I, or DIG-3 - all active against ECB).

DIG-3 is disclosed in US 2010 00269223.

Plants that produce any of the subject combinations of proteins are included within the scope of the subject invention. Additional toxins/genes can also be added, but the particular stacks discussed above advantageously and surprisingly provide multiple modes of action against FAW and/or ECB. This can help to reduce or eliminate the requirement for refuge acreage. A field thus planted of over 10 acres is thus included within the subject invention.

GENBANK can also be used to obtain the sequences for any of the genes and proteins disclosed or mentioned herein. *See* Appendix A, below.

U.S. Patent No. 5,188,960 and U.S. Patent No. 5,827,514 describe Cry1Fa core toxin containing proteins suitable for use in carrying out the present invention. U.S. Patent No. 6,218,188 describes plant-optimized DNA sequences encoding Cry1Fa core toxin-containing proteins that are suitable for use in the present invention.

Cry1Fa is in the Herculex®, SmartStax™, and WidesStrike™ products. A vip3Ab gene could be combined into, for example, a Cry1Fa product such as Herculex®, SmartStax™, and WideStrike™. Accordingly, use of Vip3Ab could be significant in reducing the selection pressure on these and other commercialized proteins. Vip3Ab could thus be used as in the 3 gene combination for corn and other plants (cotton and soybeans, for example).

Combinations of proteins described herein can be used to control lepidopteran pests. Adult lepidopterans, for example, butterflies and moths, primarily feed on flower nectar and are a significant effector of pollination. Nearly all lepidopteran larvae, *i.e.,* caterpillars, feed on plants, and many are serious pests. Caterpillars feed on or inside foliage or on the roots or stem of a plant, depriving the plant of nutrients and often destroying the plant's physical support structure. Additionally, caterpillars feed on fruit, fabrics, and stored grains and flours, ruining these products for sale or severely diminishing their value. As used herein, reference to lepidopteran pests refers to various life stages of the pest, including larval stages.

Some chimeric toxins comprise a full N-terminal core toxin portion of a *Bt* toxin and, at some point past the end of the core toxin portion, the protein has a transition to a heterologous protoxin sequence. The N-terminal, insecticidally active, toxin portion of a *Bt* toxin is referred to as the "core" toxin. The transition from the core toxin segment to the heterologous protoxin segment can occur at approximately the toxin/protoxin junction or, in the alternative, a portion of the native protoxin (extending past the core toxin portion) can be retained, with the transition to the heterologous protoxin portion occurring downstream.

As an example, one chimeric toxin is a full core toxin portion of Cry1Fa (roughly the first 600 amino acids) and a heterologous protoxin (the remainder of the protein to the C-terminus). The portion of a chimeric toxin comprising the protoxin is derived from a Cry1Ab protein toxin or the portion of a chimeric toxin comprising the protoxin is derived from a Cry1Ab protein toxin.

A person skilled in this art will appreciate that *Bt* toxins, even within a certain class such as Cry1F, will vary to some extent in length and the precise location of the transition from core toxin portion to protoxin portion. Typically, the Cry1Fa toxins are about 1150 to about 1200 amino acids in length. The transition from core toxin portion to protoxin portion will typically occur at between about 50% to about 60% of the full length toxin. The chimeric toxin will include the full expanse of this N-terminal core toxin portion. Thus, the chimeric toxin will comprise at least about 50% of the full length of the Cry1Fa *Bt* toxin protein. This will typically be at least about 590 amino acids. With regard to the protoxin portion, the full expanse of the Cry1Ab protoxin portion extends from the end of the core toxin portion to the C-terminus of the molecule.

Genes and toxins. The genes and toxins include not only the full length sequences disclosed but also fragments of these sequences, variants, mutants, and fusion proteins which retain the characteristic pesticidal activity of the toxins specifically exemplified herein. As used herein, the terms "variants" or "variations" of genes refer to nucleotide sequences which encode the same toxins or which encode equivalent toxins having pesticidal activity. As used herein, the term "equivalent toxins" refers to toxins having the same or essentially the same biological activity against the target pests as the claimed toxins.

As used herein, the boundaries represent approximately 95% (Cry1Fa's and Vip3Ab's), 78% (Cry1F's and Vip3A's), and 45% (Cry1's and Vip3's) sequence identity, per "Revision of the Nomenclature for the Bacillus thuringiensis Pesticidal Crystal Proteins," N. Crickmore, D.R. Zeigler, J. Feitelson, E. Schnepf, J. Van Rie, D. Lereclus, J. Baum, and D.H. Dean. Microbiology and Molecular Biology Reviews (1998) Vol 62: 807-813. These cut offs can also be applied to the core toxins only (for Cry1Fa, for example).

It should be apparent to a person skilled in this art that genes encoding active toxins can be identified and obtained through several means. The specific genes or gene portions exemplified herein may be obtained from the isolates deposited at a culture depository. These genes, or portions or variants thereof, may also be constructed synthetically, for example, by use of a gene synthesizer. Variations of genes may be readily constructed using standard techniques for making point mutations. Also, fragments of these genes can be made using commercially available exonucleases or endonucleases according to standard procedures. For example, enzymes such as Bal31 or site-directed mutagenesis can be used to systematically cut off nucleotides from the ends of these genes. Genes that encode active fragments may also be obtained using a variety of restriction enzymes. Proteases may be used to directly obtain active fragments of these protein toxins.

Fragments and equivalents which retain the pesticidal activity of the exemplified toxins are disclosed. Also, because of the redundancy of the genetic code, a variety of different DNA sequences can encode the amino acid sequences disclosed herein. It is well within the skill of a person trained in the art to create these alternative DNA sequences encoding the same, or essentially the same, toxins. As used herein, reference to "essentially the same" sequence refers to sequences which have amino acid substitutions, deletions, additions, or insertions which do not materially affect pesticidal activity. Fragments of genes encoding proteins that retain pesticidal activity are also included in this definition.

A further method for identifying the genes encoding the toxins and gene portions useful according to the subject invention is through the use of oligonucleotide probes. These probes are detectable nucleotide sequences. These sequences may be detectable by virtue of an appropriate label or may be made inherently fluorescent as described in International Application No. WO93/16094. As is well known in the art, if the probe molecule and nucleic acid sample hybridize by forming a strong bond between the two molecules, it can be reasonably assumed that the probe and sample have substantial homology. Preferably, hybridization is conducted under stringent conditions by techniques well-known in the art, as described, for example, in Keller, G. H., M. M. Manak (1987) DNA Probes, Stockton Press, New York, N.Y., pp. 169-170. Some examples of salt concentrations and temperature combinations are as follows (in order of increasing stringency): 2X SSPE or SSC at room temperature; 1X SSPE or SSC at 42° C; 0.1X SSPE or SSC at 42° C; 0.1X SSPE or SSC at 65° C. Detection of the probe provides a means for determining in a known manner whether hybridization has occurred. Such a probe analysis provides a rapid method for identifying toxin-encoding genes. The nucleotide segments which are used as probes can be synthesized using a DNA synthesizer and standard procedures. These nucleotide sequences can also be used as PCR primers to amplify genes.

Variant toxins. Certain toxins of the subject invention have been specifically exemplified herein. Equivalent toxins will have amino acid homology with an exemplified toxin. This amino acid homology will typically be greater than 75%, preferably be greater than 90%, and most preferably be greater than 95%. The amino acid homology will be highest in critical regions of the toxin which account for biological activity or are involved in the determination of three-dimensional configuration which ultimately is responsible for the biological activity. In this regard, certain amino acid substitutions are acceptable and can be expected if these substitutions are in regions which are not critical to activity or are conservative amino acid substitutions which do not affect the three-dimensional configuration of the molecule. For example, amino acids may be placed in the following classes: non-polar, uncharged polar, basic, and acidic. Conservative substitutions whereby an amino acid of one class is replaced with another amino acid of the same type fall within the scope of the subject invention so long as the substitution does not materially alter the biological activity of the compound. Below is a listing of examples of amino acids belonging to each class.

Table 1

| Class of Amino Acid | Examples of Amino Acids |
| --- | --- |

In some instances, non-conservative substitutions can also be made. The critical factor is that these substitutions must not significantly detract from the biological activity of the toxin.

Recombinant hosts. The genes encoding the toxins can be introduced into a wide variety of microbial or plant hosts. Expression of the toxin gene results, directly or indirectly, in the intracellular production and maintenance of the pesticide. Conjugal transfer and recombinant transfer can be used to create a Bt strain that expresses both toxins. Other host organisms may also be transformed with one or both of the toxin genes then used to accomplish the synergistic effect. With suitable microbial hosts, e.g., Pseudomonas, the microbes can be applied to the situs of the pest, where they will proliferate and be ingested. The result is control of the pest. Alternatively, the microbe hosting the toxin gene can be treated under conditions that prolong the activity of the toxin and stabilize the cell. The treated cell, which retains the toxic activity, then can be applied to the environment of the target pest.

Where the Bt toxin gene is introduced via a suitable vector into a microbial host, and said host is applied to the environment in a living state, it is essential that certain host microbes be used. Microorganism hosts are selected which are known to occupy the "phytosphere" (phylloplane, phyllosphere, rhizosphere, and/or rhizoplane) of one or more crops of interest. These microorganisms are selected so as to be capable of successfully competing in the particular environment (crop and other insect habitats) with the wild-type microorganisms, provide for stable maintenance and expression of the gene expressing the polypeptide pesticide, and, desirably, provide for improved protection of the pesticide from environmental degradation and inactivation.

A large number of microorganisms are known to inhabit the phylloplane (the surface of the plant leaves) and/or the rhizosphere (the soil surrounding plant roots) of a wide variety of important crops. These microorganisms include bacteria, algae, and fungi. Of particular interest are microorganisms, such as bacteria, e.g., genera Pseudomonas, Erwinia, Serratia, Klebsiella, Xanthomonas, Streptomyces, Rhizobium, Rhodopseudomonas, Methylophilius, Agrobactenum, Acetobacter, Lactobacillus, Arthrobacter, Azotobacter, Leuconostoc, and Alcaligenes; fungi, particularly yeast, e.g., genera Saccharomyces, Cryptococcus, Kluyveromyces, Sporobolomyces, Rhodotorula, and Aureobasidium. Of particular interest are such phytosphere bacterial species as Pseudomonas syringae, Pseudomonas fluorescens, Serratia marcescens, Acetobacter xylinum, Agrobacterium tumefaciens, Rhodopseudomonas spheroides, Xanthomonas campestris, Rhizobium melioti, Alcaligenes entrophus, and Azotobacter vinlandii; and phytosphere yeast species such as *Rhodotorula rubra, R. glutinis, R. marina, R. aurantiaca, Cryptococcus albidus, C. diffluens, C. laurentii, Saccharomyces rosei, S. pretoriensis, S. cerevisiae, Sporobolomyces roseus, S. odorus, Kluyveromyces veronae,* and *Aureobasidium pollulans.* Of particular interest are the pigmented microorganisms.

A wide variety of methods is available for introducing a *Bt* gene encoding a toxin into a microorganism host under conditions which allow for stable maintenance and expression of the gene. These methods are well known to those skilled in the art and are described, for example, in US Pat. No. 5135867.

Treatment of cells. *Bacillus thuringiensis* or recombinant cells expressing the *Bt* toxins can be treated to prolong the toxin activity and stabilize the cell. The pesticide microcapsule that is formed comprises the *Bt* toxin or toxins within a cellular structure that has been stabilized and will protect the toxin when the microcapsule is applied to the environment of the target pest. Suitable host cells may include either prokaryotes or eukaryotes, normally being limited to those cells which do not produce substances toxic to higher organisms, such as mammals. However, organisms which produce substances toxic to higher organisms could be used, where the toxic substances are unstable or the level of application sufficiently low as to avoid any possibility of toxicity to a mammalian host. As hosts, of particular interest will be the prokaryotes and the lower eukaryotes, such as fungi.

The cell will usually be intact and be substantially in the proliferative form when treated, rather than in a spore form, although in some instances spores may be employed.

Treatment of the microbial cell, e.g., a microbe containing the B.t. toxin gene or genes, can be by chemical or physical means, or by a combination of chemical and/or physical means, so long as the technique does not deleteriously affect the properties of the toxin, nor diminish the cellular capability of protecting the toxin. Examples of chemical reagents are halogenating agents, particularly halogens of atomic no. 17-80. More particularly, iodine can be used under mild conditions and for sufficient time to achieve the desired results. Other suitable techniques include treatment with aldehydes, such as glutaraldehyde; anti-infectives, such as zephiran chloride and cetylpyridinium chloride; alcohols, such as isopropyl and ethanol; various histologic fixatives, such as Lugol iodine, Bouin's fixative, various acids and Helly's fixative (See: Humason, Gretchen L., Animal Tissue Techniques, W. H. Freeman and Company, 1967); or a combination of physical (heat) and chemical agents that preserve and prolong the activity of the toxin produced in the cell when the cell is administered to the host environment. Examples of physical means are short wavelength radiation such as gamma-radiation and X-radiation, freezing, UV irradiation, lyophilization, and the like. Methods for treatment of microbial cells are disclosed in U.S. Pat. Nos. 4,695,455 and 4,965,462.

The cells generally will have enhanced structural stability which will enhance resistance to environmental conditions. Where the pesticide is in a proform, the method of cell treatment should be selected so as not to inhibit processing of the proform to the mature form of the pesticide by the target pest pathogen. For example, formaldehyde will crosslink proteins and could inhibit processing of the proform of a polypeptide pesticide. The method of treatment should retain at least a substantial portion of the bio-availability or bioactivity of the toxin.

Characteristics of particular interest in selecting a host cell for purposes of production include ease of introducing the B.t. gene or genes into the host, availability of expression systems, efficiency of expression, stability of the pesticide in the host, and the presence of auxiliary genetic capabilities. Characteristics of interest for use as a pesticide microcapsule include protective qualities for the pesticide, such as thick cell walls, pigmentation, and intracellular packaging or formation of inclusion bodies; survival in aqueous environments; lack of mammalian toxicity; attractiveness to pests for ingestion; ease of killing and fixing without damage to the toxin; and the like. Other considerations include ease of formulation and handling, economics, storage stability, and the like.

Growth of cells. The cellular host containing the B.t. insecticidal gene or genes may be grown in any convenient nutrient medium, where the DNA construct provides a selective advantage, providing for a selective medium so that substantially all or all of the cells retain the B.t. gene. These cells may then be harvested in accordance with conventional ways. Alternatively, the cells can be treated prior to harvesting.

The B.t. cells producing the toxins of the invention can be cultured using standard art media and fermentation techniques. Upon completion of the fermentation cycle the bacteria can be harvested by first separating the B.t. spores and crystals from the fermentation broth by means well known in the art. The recovered B.t. spores and crystals can be formulated into a wettable powder, liquid concentrate, granules or other formulations by the addition of surfactants, dispersants, inert carriers, and other components to facilitate handling and application for particular target pests. These formulations and application procedures are all well known in the art.

Formulations. Formulated bait granules containing an attractant and spores, crystals, and toxins of the B.t. isolates, or recombinant microbes comprising the genes obtainable from the B.t. isolates disclosed herein, can be applied to the soil. Formulated product can also be applied as a seed-coating or root treatment or total plant treatment at later stages of the crop cycle. Plant and soil treatments of B.t. cells may be employed as wettable powders, granules or dusts, by mixing with various inert materials, such as inorganic minerals (phyllosilicates, carbonates, sulfates, phosphates, and the like) or botanical materials (powdered corncobs, rice hulls, walnut shells, and the like). The formulations may include spreader-sticker adjuvants, stabilizing agents, other pesticidal additives, or surfactants. Liquid formulations may be aqueous-based or non-aqueous and employed as foams, gels, suspensions, emulsifiable concentrates, or the like. The ingredients may include rheological agents, surfactants, emulsifiers, dispersants, or polymers.

As would be appreciated by a person skilled in the art, the pesticidal concentration will vary widely depending upon the nature of the particular formulation, particularly whether it is a concentrate or to be used directly. The pesticide will be present in at least 1% by weight and may be 100% by weight. The dry formulations will have from about 1-95% by weight of the pesticide while the liquid formulations will generally be from about 1-60% by weight of the solids in the liquid phase. The formulations will generally have from about $10^2$ to about $10^4$ cells/mg. These formulations will be administered at about 50 mg (liquid or dry) to 1 kg or more per hectare.

The formulations can be applied to the environment of the lepidopteran pest, e.g., foliage or soil, by spraying, dusting, sprinkling, or the like.

Plant transformation. A preferred recombinant host for production of the insecticidal proteins of the subject invention is a transformed plant. Genes encoding *Bt* toxin proteins, as disclosed herein, can be inserted into plant cells using a variety of techniques which are well known in the art. For example, a large number of cloning vectors comprising a replication system in *Escherichia coli* and a marker that permits selection of the transformed cells are available for preparation for the insertion of foreign genes into higher plants. The vectors comprise, for example, pBR322, pUC series, M13mp series, pACYC184, *inter alia.* Accordingly, the DNA fragment having the sequence encoding the *Bt* toxin protein can be inserted into the vector at a suitable restriction site. The resulting plasmid is used for transformation into *E. coli.* The *E. coli* cells are cultivated in a suitable nutrient medium, then harvested and lysed. The plasmid is recovered. Sequence analysis, restriction analysis, electrophoresis, and other biochemical-molecular biological methods are generally carried out as methods of analysis. After each manipulation, the DNA sequence used can be cleaved and joined to the next DNA sequence. Each plasmid sequence can be cloned in the same or other plasmids. Depending on the method of inserting desired genes into the plant, other DNA sequences may be necessary. If, for example, the Ti or Ri plasmid is used for the transformation of the plant cell, then at least the right border, but often the right and the left border of the Ti or Ri plasmid T-DNA, has to be joined as the flanking region of the genes to be inserted. The use of T-DNA for the transformation of plant cells has been intensively researched and sufficiently described in EP 120 516, Lee and Gelvin (2008), Hoekema (1985), Fraley *et al.,* (1986), and An *et al.,* (1985), and is well established in the art.

Once the inserted DNA has been integrated in the plant genome, it is relatively stable. The transformation vector normally contains a selectable marker that confers on the transformed plant cells resistance to a biocide or an antibiotic, such as Bialaphos, Kanamycin, G418, Bleomycin, or Hygromycin, *inter alia.* The individually employed marker should accordingly permit the selection of transformed cells rather than cells that do not contain the inserted DNA.

A large number of techniques are available for inserting DNA into a plant host cell. Those techniques include transformation with T-DNA using *Agrobacterium tumefaciens* or *Agrobacterium rhizogenes* as transformation agent, fusion, injection, biolistics (microparticle bombardment), or electroporation as well as other possible methods. If Agrobacteria are used for the transformation, the DNA to be inserted has to be cloned into special plasmids, namely either into an intermediate vector or into a binary vector. The intermediate vectors can be integrated into the Ti or Ri plasmid by homologous recombination owing to sequences that are homologous to sequences in the T-DNA. The Ti or Ri plasmid also comprises the vir region necessary for the transfer of the T-DNA. Intermediate vectors cannot replicate themselves in Agrobacteria. The intermediate vector can be transferred into *Agrobacterium tumefaciens* by means of a helper plasmid (conjugation). Binary vectors can replicate themselves both in *E. coli* and in Agrobacteria. They comprise a selection marker gene and a linker or polylinker which are framed by the Right and Left T-DNA border regions. They can be transformed directly into Agrobacteria (Holsters *et al.,* 1978). The *Agrobacterium* used as host cell is to comprise a plasmid carrying a vir region. The vir region is necessary for the transfer of the T-DNA into the plant cell. Additional T-DNA may be contained. The bacterium so transformed is used for the transformation of plant cells. Plant explants can advantageously be cultivated with *Agrobacterium tumefaciens* or *Agrobacterium rhizogenes* for the transfer of the DNA into the plant cell. Whole plants can then be regenerated from the infected plant material (for example, pieces of leaf, segments of stalk, roots, but also protoplasts or suspension-cultivated cells) in a suitable medium, which may contain antibiotics or biocides for selection. The plants so obtained can then be tested for the presence of the inserted DNA. No special demands are made of the plasmids in the case of injection and electroporation. It is possible to use ordinary plasmids, such as, for example, pUC derivatives.

The transformed cells grow inside the plants in the usual manner. They can form germ cells and transmit the transformed trait(s) to progeny plants. Such plants can be grown in the normal manner and crossed with plants that have the same transformed hereditary factors or other hereditary factors. The resulting hybrid individuals have the corresponding phenotypic properties.

In a preferred embodiment of the subject invention, plants will be transformed with genes wherein the codon usage has been optimized for plants. See, for example, US Patent No. 5380831. While some truncated toxins are exemplified herein, it is well-known in the *Bt* art that 130 kDa-type (full-length) toxins have an N-terminal half that is the core toxin, and a C-terminal half that is the protoxin "tail." Thus, appropriate "tails" can be used with truncated / core toxins of the subject invention. *See e.g.* US Patent No. 6218188 and US Patent No. 6673990. In addition, methods for creating synthetic *Bt* genes for use in plants are known in the art (Stewart and Burgin, 2007). One non-limiting example of a preferred transformed plant is a fertile maize plant comprising a plant expressible gene encoding a Cry1Fa protein, and further comprising a second plant expressible gene encoding a Vip3Ab protein.

Transfer (or introgression) of the Cry1Fa- and Vip3Ab-determined trait(s) into inbred maize lines can be achieved by recurrent selection breeding, for example by backcrossing. In this case, a desired recurrent parent is first crossed to a donor inbred (the non-recurrent parent) that carries the appropriate gene(s) for the Cry1F- and Vip3Ab-determined traits. The progeny of this cross is then mated back to the recurrent parent followed by selection in the resultant progeny for the desired trait(s) to be transferred from the non-recurrent parent. After three, preferably four, more preferably five or more generations of backcrosses with the recurrent parent with selection for the desired trait(s), the progeny will be heterozygous for loci controlling the trait(s) being transferred, but will be like the recurrent parent for most or almost all other genes (see, for example, Poehlman & Sleper (1995) Breeding Field Crops, 4th Ed., 172-175; Fehr (1987) Principles of Cultivar Development, Vol. 1: Theory and Technique, 360-376).

Insect Resistance Management (IRM) Strategies. Roush et al., for example, outlines two-toxin strategies, also called "pyramiding" or "stacking," for management of insecticidal transgenic crops. (The Royal Society. Phil. Trans. R. Soc. Lond. B. (1998) 353, 1777-1786).

On their website, the United States Environmental Protection Agency (epa.gov/oppbppd1/biopesticides/pips/bt_corn_refuge_2006.htm) publishes the following requirements for providing non-transgenic (*i.e., non-B.t.*) refuges (a section of non-Bt crops / corn) for use with transgenic crops producing a single Bt protein active against target pests.

"The specific structured requirements for corn borer-protected Bt (Cry1Ab or Cry1F) corn products are as follows:

Structured refuges:  20% non-Lepidopteran Bt corn refuge in Corn Belt;

50% non-Lepidopteran Bt refuge in Cotton Belt

Blocks

Internal (*i.e.,* within the Bt field)
External (*i.e.,* separate fields within ½ mile (¼ mile if possible) of the
Bt field to maximize random mating)

In-field Strips

Strips must be at least 4 rows wide (preferably 6 rows) to reduce
the effects of larval movement"

In addition, the National Corn Growers Association, on their website:
(ncga.com/insect-resistance-management-fact-sheet-bt-corn)

also provides similar guidance regarding the refuge requirements. For example:

"Requirements of the Corn Borer IRM:

- Plant at least 20% of your corn acres to refuge hybrids
- In cotton producing regions, refuge must be 50%
- Must be planted within 1/2 mile of the refuge hybrids
- Refuge can be planted as strips within the Bt field; the refuge strips must be at least 4 rows wide
- Refuge may be treated with conventional pesticides only if economic thresholds are reached for target insect
- Bt-based sprayable insecticides cannot be used on the refuge corn
- Appropriate refuge must be planted on every farm with Bt corn"

As stated by Roush *et al.* (on pages 1780 and 1784 right column, for example), stacking or pyramiding of two different proteins each effective against the target pests and with little or no cross-resistance can allow for use of a smaller refuge. Roush suggests that for a successful stack, a refuge size of less than 10% refuge, can provide comparable resistance management to about 50% refuge for a single (non-pyramided) trait. For currently available pyramided Bt corn products, the U.S. Environmental Protection Agency requires significantly less (generally 5%) structured refuge of non-Bt corn be planted than for single trait products (generally 20%).

There are various ways of providing the IRM effects of a refuge, including various geometric planting patterns in the fields (as mentioned above) and in-bag seed mixtures, as discussed further by Roush *et al.* (*supra*), and U.S.

Patent No. 6,551,962.

The above percentages, or similar refuge ratios, can be used for the subject double or triple stacks or pyramids. For triple stacks with three modes of action against a single target pest, a goal would be zero refuge (or less than 5% refuge, for example). This is particularly true for commercial acreage - of over 10 acres for example.

Unless specifically indicated or implied, the terms "a", "an", and "the" signify "at least one" as used herein.

Following are examples that illustrate procedures for practicing the invention. These examples should not be construed as limiting. All percentages are by weight and all solvent mixture proportions are by volume unless otherwise noted. All temperatures are in degrees Celsius.

EXAMPLES

## Example 1 - Summary Of Examples

Examples are given showing that Vip3Ab1 is active against *Spodoptera frugiperda* (fall armyworm) wild type larvae, and against a field collected strain of *Spodoptera frugiperda* found in Puerto Rico that is resistant to the *Bacillus thuringiensis* crystal toxin Cry1Fa. This biological data supports the utility of Vip3Ab1 to be used to combat the development of Cry1 resistance in insects, since insects developing resistance to the Cry1Fa toxins would continue to be susceptible to the toxicity of Vip3Ab1.

Similarly, in *Spodoptera frugiperda,* $^{125}$I radiolabeled Cry1Fa binds to receptor proteins and the binding can be displaced using non-radiolabeled Cry1Fa. However, Vip3Ab1 cannot displace the binding of $^{125}$I Cry1Fa from its receptor in these experiments. These results indicate that Vip3Ab1 has a unique binding site as compared to Cry1Fa. The ability of Vip3Ab1 to exert toxicity against insects that are resistant to Cry1Fa stems from its demonstrated non-interaction at the site where these toxins bind. Further data is presented that shows the nature of Cry1Fa resistance in *Spodoptera frugiperda* is due to the inability of Cry1Fa to bind to BBMV's prepared from this insect. The biological activity of Vip3Ab1 against Cry1Fa resistant *S. frugiperda* larvae that lost their ability to bind Cry1Fa, further supports the non-interacting target site of Vip3Ab1 as compared to Cry1Fa.

## Example 2 - Purification and trypsin processing of Cry1Fa and Vip3Ab1 proteins.

The genes encoding the Cry1Fa and Vip3Ab1 pro toxins were expressed in *Pseudomonas fluorescens* expression strains and the full length proteins isolated as insoluble inclusion bodies. The washed inclusion bodies were solubilized by stirring at 37 °C in buffer containing 20 mM CAPS buffer, pH 11, + 10 mM DDT, + 0.1% 2-mercaptoethanol, for 2 hrs. The solution was centrifuged at 27,000 x g for 10 min. at 37 °C and the supernatant treated with 0.5% (w/v) TCPK treated trypsin (Sigma). This solution was incubated with mixing for an additional 1 hr. at room temperature, filtered, then loaded onto a Pharmacia Mono Q 1010 column equilibrated with 20 mM CAPS pH 10.5. After washing the loaded column with 2 column volumes of buffer, the truncated toxin was eluted using a linear gradient of 0 to 0.5 M NaCl in 20 mM CAPS in 15 column volumes at a flow rate of 1.0 ml/min. Purified trypsin truncated Cry proteins eluted at about 0.2-0.3 M NaCl. The purity of the proteins was checked by SDS PAGE and with visualization using Coomassie brilliant blue dye. In some cases, the combined fractions of the purified toxin were concentrated and loaded onto a Superose 6 column (1.6 cm dia., 60 cm long), and further purified by size exclusion chromatography. Fractions comprising a single peak of the monomeric molecular weight were combined, and concentrated, resulting in a preparation more than 95% homogeneous for a protein having a molecular weight of about 60,000 kDa.

Processing of Vip3Ab1 was achieved in a similar manner starting with the purified full length 85 kDa protein (DIG-307) provided by Monte Badger. The protein (12 mg) was dialyzed into 50 mM sodium phosphate buffer, pH 8.4, then processed by adding 1 mg of solid trypsin and incubating for 1 hrs. at room temperature. The solution was loaded onto a MonoQ anion exchange column (1 cm dia., 10 cm. long), and eluted with a linear gradient of NaCl from 0 to 500 mM in 20 mM sodium phosphate buffer, pH 8.4 over 7 column volumes. Elution of the protein was monitored by SDS-PAGE. The major processed band had a molecular weight of 65 kDa, as determined by SDS-PAGE using molecular weight standards for comparison.

## Example 3 - Insect Bioassays.

Purified proteins were tested for insecticidal activity in bioassays conducted with neonate *Spodoptera frugiperda* (J.E. Smith) larvae on artificial insect diet. The Cry1F-resistant FAW were collected from fields of Herculex I (Cry1Fa) corn in Puerto Rico, and brought into the Dow AgroSciences Insectary for continuous rearing. Characterization of this strain of resistant-FAW is outlined in the internal report by Schlenz, et al (Schlenz et al., 2008).

Insect bioassays were conducted in 128-well plastic bioassay trays (C-D International, Pitman, NJ). Each well contained 0.5 mL of multi-species lepidoptera diet (Southland Products, Lake Village, AR). A 40 $\mu$L aliquot of the purified Cry or Vip3Ab1 protein diluted to various concentrations in 10 mM CAPS, pH 10.5, or control solution was delivered by pipette onto the 1.5 cm$^2$ diet surface of each well (26.7 $\mu$L/cm$^2$). Sixteen wells were tested per sample. The negative control was a buffer solution blank containing no protein. Positive controls included preparations of Cry1F. The treated trays were held in a fume hood until the liquid on the diet surface had evaporated or was absorbed into the diet.

Within a few hours of eclosion, individual larvae were picked up with a moistened camelhair brush and deposited on the treated diet, one larva per well. The infested wells were then sealed with adhesive sheets of clear plastic that are vented to allow gas exchange (C-D International, Pitman, NJ). The bioassay trays were held under controlled environmental conditions (28°C, ~40% RH, 16:8 [L:D] photoperiod). After 5 days, the total number of insects exposed to each protein sample, the number of dead insects, and the weight of surviving insects were recorded.

## Example 4 - Iodination of Cry1Fa toxins.

Iodination of Cry1F has been reported to destroy both the toxicity and the binding capacity of this protein when tested against tobacco budworm larvae and BBMV's prepared from these insects (Luo et al., 1999; Sheets and Storer, 2001). The inactivation is presumably due to the need for unmodified tyrosine residues near its binding site. When Cry1F was iodinated using the Iodo-bead method, the protein lost all of its ability to exhibit specific binding characteristics using BBMV's from *H. virescens.* Using non-radiolabeled NaI to iodinate Cry1F employing the Iodo-bead method, the iodinated Cry1F also lost its insecticidal activity against *H. virescens.*

Earlier studies in our laboratories demonstrated that Cry1Fa could be fluorescently labeled using maleimide conjugated labeling reagents that specifically alkylate proteins at cysteine residues. Since the Cry1Fa trypsin core toxin contains a single cysteine residue at position 205, labeling the protein with such a reagent would result in alkylation of the protein at a single specific site. It was determined that Cry1Fa could be fluorescently labeled with fluorescein-5-maleimide and that the labeled protein retained insecticidal activity. Based upon the retention of biological activity of the cysteine fluorescein labeled Cry1Fa, it was determined that we could also radioiodinate the fluorescein portion of the label by the method of Palmer et al.,(Palmer et al., 1997), and attach it to the cysteine of Cry1Fa and have a radiolabeled Cry1Fa that retains biological activity.

Fluorescein-5-maleimide was dissolved to 10 mM (4.27 mg/ml) in DMSO, then diluted to 1 mM in PBS as determined by its molar extinction coefficient of 68,000 M$^{-1}$cm$^{-1}$. To a 70 $\mu$l solution of PBS containing two Iodobeads, 0.5 mCi of Na$^{125}$I was added behind lead shielding. The solution was allowed to mix at room temperature for 5 min., then 10 $\mu$l of the 1 mM fluorescein-5-maleimide was added. The reactants were allowed to react for 10 min., and then removed from the iodobeads. To the reacted solution was added 2 $\mu$g of highly purified trypsin truncated Cry1Fa core toxin in PBS. The protein was incubated with the iodinated fluorescein-5-maleimide solution for 48 hrs at 4 °C. The reaction was stopped by adding 2-mercapto ethanol to 14 mM. The reaction mixture was then added to a Zebra spin column equilibrated in 20 mM CAPS, 150 mM KCl, pH 9, and centrifuged at 1,500 x g for 2 min. to separate non-reacted iodinated dye from the protein. The $^{125}$I radiolabeled fluorescein-Cry1Fa was counted in a gamma counter to determine its specific activity determined based upon an assumed 80% recovery of the input toxin. The protein was also characterized by SDS-PAGE and visualized by phosphor imaging to assure that the radioactivity measured was covalently associated with the Cry1Fa protein.

## Example 5 - Preparation and Fractionation of Solubilized BBMV's.

Standard methods of protein quantification and SDS-polyacrylamide gel electrophoresis were employed as taught, for example, in Sambrook et al. (Sambrook and Russell, 2001) and updates thereof. Last instar *S. frugiperda* larvae were fasted overnight and then dissected after chilling on ice for 15 minutes. The midgut tissue was removed from the body cavity, leaving behind the hindgut attached to the integument. The midgut was placed in a 9X volume of ice cold homogenization buffer (300 mM mannitol, 5 mM EGTA, 17 mM Tris base, pH7.5), supplemented with Protease Inhibitor Cocktail (Sigma-Aldrich P-2714) diluted as recommended by the supplier. The tissue was homogenized with 15 strokes of a glass tissue homogenizer. BBMV's were prepared by the MgCl$_2$ precipitation method of Wolfersberger (Wolfersberger, 1993). Briefly, an equal volume of a 24 mM MgCl$_2$ solution in 300 mM mannitol was mixed with the midgut homogenate, stirred for 5 minutes and allowed to stand on ice for 15 min. The solution was centrifuged at 2,500 x g for 15 min at 4°C. The supernatant was saved and the pellet suspended into the original volume of 0.5X diluted homogenization buffer and centrifuged again. The two supernatants were combined and centrifuged at 27,000 x g for 30 min at 4°C to form the BBMV fraction. The pellet was suspended into BBMV Storage Buffer (10 mM HEPES, 130 mM KCl, 10% glycerol, pH 7.4) to a concentration of about 3 mg/ml protein. Protein concentration was determined using BSA as the standard.

L-leucine-p-nitroanilide aminopeptidase activity (a marker enzyme for the BBMV fraction) was determined prior to freezing the samples. Briefly, 50 $\mu$l of L-leucine-p-nitroanilide (1 mg/ml in PBS) was added to 940 ml 50 mM Tris HCl in a standard cuvette. The cuvette was placed in a Cary 50 Bio spectrophotometer, zeroed for absorbance reading at 405 nm, and the reaction initiated by adding 10 μl of either insect midgut homogenate or insect BBMV preparation. The increase in absorbance at 405 nm was monitored for 5 minutes at room temperature. The specific activity of the homogenate and BBMV preparations was determined based upon the kinetics of the absorbance increase over time during a linear increase in absorbance per unit total protein added to the assay based upon the following equation:

$$\Delta OD/(min*mg) = \text{Aminopeptidase Rate } (\Delta OD/ml*min)/ [protein] (mg/ml)$$

The specific activity of this enzyme typically increased 7-fold compared to that found in the starting midgut homogenate fraction. The BBMV's were aliquoted into 250 μl samples, flash frozen in liquid $N_2$ and stored at -80°C.

**Example 6 - Electrophoresis.**

Analysis of proteins by SDS-PAGE was conducted under reducing (i.e. in 5% β-mercaptoethanol, BME) and denaturing (i.e. heated 5 minutes at 90° in the presence of 4% SDS) conditions. Proteins were loaded into wells of a 4% to 20% tris-glycine polyacrylamide gel (BioRad; Hercules, CA) and separated at 200 volts for 60 minutes. Protein bands were detected by staining with Coomassie Brilliant Blue R-250 (BioRad) for one hour, and destained with a solution of 5% methanol in 7% acetic acid. The gels were imaged and analyzed using a BioRad Fluro-S Multi Imager™. Relative molecular weights of the protein bands were determined by comparison to the mobilities of known molecular weight proteins observed in a sample of BenchMark™ Protein Ladder (Invitrogen, Carlsbad, CA) loaded into one well of the gel.

**Example 7 - Imaging.**

Radio-purity of the iodinated Cry proteins and measurement of radioactive Cry1Fa in pull down assays was determined by SDS-PAGE and phosphorimaging. Briefly, SDS-PAGE gels were imaged by wrapping the gels in Mylar film (12 μm thick), after separation and fixation of the protein, then exposing the gel under a Molecular Dynamics storage phosphor screen (35 cm x 43 cm) for at least overnight, and up to 4 days. The plates were developed using a Molecular Dynamics Storm 820 phosphor-imager and the image was analyzed using ImageQuant ™ software.

**Example 8 - Summary of Results**

Mortality results from bioassays of the full length Vip3Ab1 protein tested at a variety of doses against wild type and Cry1Fa resistant *S. frugiperda* larvae are shown in Figure 1. Against wild type *S. frugiperda* larvae, we obtained 100% mortality at the highest concentration tested (9,000 ng/cm$^2$), and lower levels of mortality at lower doses. The LC-50 was estimated at about 2,000 ng/cm$^2$. Vip3Ab1 was highly effective against *S. frugiperda* in inhibiting growth of the larvae, with greater than 95% growth inhibition at concentrations of 1,000 ng/cm$^2$ and higher. The high level of growth inhibition observed for both *S. frugiperda* larvae suggests that these insects would most likely progress to mortality if left for a longer time period.

A bioassay was also conducted to compare the biological activity of Vip3Ab1 against wild type *S. frugiperda* versus Cry1Fa resistant *S. frugiperda* (Figure 1). Percent growth inhibition is indicated by the vertical bars, and percent mortality by the diamond symbols. Mortality measured 5 days after exposure to the toxin was below 50% for both insect types at all concentrations tested. A clear dose response was obtained for growth inhibition. Vip3Ab1 resulted in >95% inhibition of larval growth of both Cry1Fa sensitive and Cry1Fa resistant *S. frugiperda* larvae at concentrations above 1,000 ng/cm$^2$, and resulted in about 50% inhibition of larval growth of the wild type *S. frugiperda* at approximately 40 ng/cm$^2$. Vip3Ab1 resulted in more than 50% growth inhibition of Cry1Fa resistant *S. frugiperda* at all concentrations tested, down to the lowest of 4.1 ng/cm$^2$. Thus, Vip3Ab1 has high activity against Cry1Fa resistant *S. frugiperda* larvae.

Additional bioassay replications were conducted to generate median lethal concentrations (LC50), median growth inhibition concentrations. Table 2 shows (GI50) and 95% confidence intervals of Cry1F-suseptible Spodoptera frugiperda and Cry1F-resistant Spodoptera frugiperda to Vip3Ab1 compared to controls.

Table 2

| Insect | LC-50 | 95% CI | GI-50 | 95% CI |
|---|---|---|---|---|
| **FAW** | 3966.3 | (2150.3 - 9406.6) | 21.9 | (18.5 - 25.6) |
| Cry1Fa pos ctrl vs FAW | | | | |
| **rFAW** | 499.9 | (338.9 - 748.6) | 7.7 | (5.5 - 10.7) |
| Cry1Fa pos ctrl vs rFAW | | no growth inhibition seen within each tested dose | | |
| Buffer (FAW, rFAW) | no mortality | | AVG. Wt. per insect | 63.2 mg (FAW) 35.3 mg (rFAW) |
| Water (FAW, rFAW) | no mortality | | AVG. Wt. per insect | 63.1 mg (FAW) 35.9 mg (rFAW) |

Radiolabeled competition binding assays were conducted to determine if Vip3Ab1 interacts at the same site that Cry1Fa binds in FAW. A competition assay was developed to measure the ability of Vip3Ab to compete with the binding of $^{125}$I radiolabeled Cry1Fa.

Figure 2 shows the phosphorimage of radioactive Cry1Fa separated by SDS-PAGE after binding to BBMV proteins. In the absence of any competing ligands, $^{125}$I Cry1Fa can be detected associated with the BBMV protein. When incubated in the presence of 1,000 nM unlabeled Cry1Fa (500-fold excess compared to the concentration of labeled protein used in the assay), very little radioactivity is detected corresponding to $^{125}$I Cry1Fa. Thus, this result shows that the unlabeled Cry1Fa effectively competes with the radiolabeled Cry1Fa for binding to the receptor proteins, as would be expected since these homologous proteins bind to the same site. When the same experiment is conducted using 1,000 nM unlabeled Vip3Ab1 protein as the competing protein, we see no change in the level of $^{125}$I Cry1Fa binding to the BBMV proteins from *S. frugiperda,* indicating that Vip3Ab1 does not compete with the binding of $^{125}$I Cry1Fa. This result is interpreted to indicate that Vip3Ab1 does not bind at the same site as Cry1Fa.

Insects can develop resistance to the toxicity of Cry proteins through a number of different biochemical mechanisms, but the most common mechanism is due to a reduction in the ability of the Cry toxin protein to bind to its specific receptor in the gut of the insect (Heckel et al., 2007; Tabashnik et al., 2000; Xu et al., 2005). This can be brought about thought small point mutations, large gene deletions, or though other genetic or biochemical mechanisms. When we investigated the BBMV proteins from Cry1Fa resistant *S. frugiperda* to understand the nature of their resistance to Cry1Fa, we discovered that BBMV's prepared from Cry1Fa resistant insects were much less able to bind $^{125}$I radiolabeled Cry1Fa as compared to BBMV's prepared from the wild type insects (Figure 3). Thus, the mechanism of resistance to Cry1Fa in *S. frugiperda* is due to a greatly reduced level of binding of Cry1Fa to the BBMV's from the resistant insects. Since we show in Figure 2 that Vip3Ab1 does not compete with the binding of Cry1Fa, this further demonstrates that the Vip3Ab1 should not be affected by a resistance mechanism that is involved with the binding of Cry1Fa to its specific receptor. This is born out in the bioassays. Thus, Vip3Ab1 complements the activity of Cry1Fa, in that it has biological activity against similar insects, yet does not bind to the same receptor sites as these Cry proteins, and thus is not affected by resistance mechanisms that would involve reduction of Cry toxin binding. We concluded from these studies that Vip3Ab1 is an excellent insect toxin to combine with Cry1Fa as an insect resistance management approach to provide biological activity against insects that may have developed resistance to either one of these proteins, and also to prevent resistant insects.

Reference List

Heckel,D.G., Gahan,L.J., Baxter,S.W., Zhao,J.Z., Shelton,A.M., Gould,F., and Tabashnik,B.E. (2007). The diversity of Bt resistance genes in species of Lepidoptera. J Invertebr Pathol 95, 192-197.

Luo,K., Banks,D., and Adang,M.J. (1999). Toxicity, binding, and permeability analyses of four bacillus thuringiensis cry1 delta-endotoxins using brush border membrane vesicles of spodoptera exigua and spodoptera frugiperda. Appl. Environ. Microbiol. 65, 457-464.

Palmer, M., Buchkremer, M, Valeva, A, and Bhakdi, S. Cysteine-specific radioiodination of proteins with fluorescein maleimide. Analytical Biochemistry 253, 175-179. 1997. Ref Type: Journal (Full)

Sambrook,J. and Russell,D.W. (2001). Molecular Cloning: A Laboratory Manual. Cold Spring Harbor Laboratory).

Schlenz, M. L., Babcock, J. M., and Storer, N. P. Response of Cry1F-resistant and Susceptible European Corn Borer and Fall Armyworm Colonies to Cry1A.105 and Cry12Ab2. DAI 0830, 2008. Indianapolis, Dow AgroSciences. Derbi Report.

Sheets, J. J. and Storer, N. P. Analysis of Cry1Ac Binding to Proteins in Brush Border Membrane Vesicles of Corn Earworm Larvae (Heleothis zea). Interactions with Cry1F Proteins and Its Implication for Resistance in the Field. DAI-0417, 1-26. 2001. Indianapolis, Dow AgroSciences.

Tabashnik,B.E., Liu,Y.B., Finson,N., Masson,L., and Heckel,D.G. (1997). One gene in diamondback moth confers resistance to four Bacillus thuringiensis toxins. Proc. Natl. Acad. Sci. U. S. A 94, 1640-1644.

Tabashnik,B.E., Malvar,T., Liu,Y.B., Finson,N., Borthakur,D., Shin,B.S., Park,S.H., Masson,L., de Maagd,R.A., and Bosch,D. (1996). Cross-resistance of the diamondback moth indicates altered interactions with domain II of Bacillus thuringiensis toxins. Appl. Environ. Microbiol. 62, 2839-2844.

Tabashnik,B.E., Roush,R.T., Earle,E.D., and Shelton,A.M. (2000). Resistance to Bt toxins. Science 287, 42.

Wolfersberger,M.G. (1993). Preparation and partial characterization of amino acid transporting brush border membrane vesicles from the larval midgut of the gypsy moth (Lymantria dispar). Arch. Insect Biochem. Physiol 24, 139-147.

Xu,X., Yu,L., and Wu,Y. (2005). Disruption of a cadherin gene associated with resistance to Cry1Ac {delta}-endotoxin of Bacillus thuringiensis in Helicoverpa armigera. Appl Environ Microbiol 71, 948-954.

**Appendix A**

**List of delta-endotoxins - from Crickmore et al. website (cited in application)**

**Accession Number is to NCBI entry**

| Name | Acc No. | Authors | Year | Source Strain | Comment |
|------|---------|---------|------|---------------|---------|
| Cry1Aa1 | AAA22353 | Schnepf et al | 1985 | Bt kurstaki HD1 | |
| Cry1Aa2 | AAA22552 | Shibano et al | 1985 | Bt sotto | |
| Cry1Aa3 | BAA00257 | Shimizu et al | 1988 | Bt aizawai IPL7 | |
| Cry1Aa4 | CAA31886 | Masson et al | 1989 | Bt entomocidus | |
| Cry1Aa5 | BAA04468 | Udayasuriyan et al | 1994 | Bt Fu-2-7 | |
| Cry1Aa6 | AAA86265 | Masson et al | 1994 | Bt kurstaki NRD-12 | |
| Cry1Aa7 | AAD46139 | Osman et al | 1999 | Bt C12 | |
| Cry1Aa8 | I26149 | Liu | 1996 | | DNA sequence only |
| Cry1Aa9 | BAA77213 | Nagamatsu et al | 1999 | Bt dendrolimus T84A1 | |
| Cry1Aa10 | AAD55382 | Hou and Chen | 1999 | Bt kurstaki HD-1-02 | |
| Cry1Aa11 | CAA70856 | Tounsi et al | 1999 | Bt kurstaki | |
| Cry1Aa12 | AAP80146 | Yao et al | 2001 | Bt Ly30 | |
| Cry1Aa13 | AAM44305 | Zhong et al | 2002 | Bt sotto | |
| Cry1Aa14 | AAP40639 | Ren et al | 2002 | unpublished | |
| Cry1Aa15 | AAY66993 | Sauka et al | 2005 | Bt INTA Mol-12 | |
| Cry1Ab1 | AAA22330 | Wabiko et al | 1986 | Bt berliner 1715 | |
| Cry1Ab2 | AAA22613 | Thorne et al | 1986 | Bt kurstaki | |
| Cry1Ab3 | AAA22561 | Geiser et al | 1986 | Bt kurstaki HD1 | |
| Cry1Ab4 | BAA00071 | Kondo et al | 1987 | Bt kurstaki HD1 | |
| Cry1Ab5 | CAA28405 | Hofte et al | 1986 | Bt berliner 1715 | |
| Cry1Ab6 | AAA22420 | Hefford et al | 1987 | Bt kurstaki NRD-12 | |

(continued)

| Name | Acc No. | Authors | Year | Source Strain | Comment |
|------|---------|---------|------|---------------|---------|
| Cry1Ab7 | CAA31620 | Haider & Ellar | 1988 | Bt aizawai IC1 | |
| Cry1Ab8 | AAA22551 | Oeda et al | 1987 | Bt aizawai IPL7 | |
| Cry1Ab9 | CAA38701 | Chak & Jen | 1993 | Bt aizawai HD133 | |
| Cry1Ab10 | A29125 | Fischhoff et al | 1987 | Bt kurstaki HD 1 | |
| Cry1Ab11 | I12419 | Ely & Tippett | 1995 | Bt A20 | DNA sequence only |
| Cry1Ab12 | AAC64003 | Silva-Werneck et al | 1998 | Bt kurstaki S93 | |
| Cry1Ab13 | AAN76494 | Tan et al | 2002 | Bt c005 | |
| Cry1Ab14 | AAG16877 | Meza-Basso & Theoduloz | 2000 | Native Chilean Bt | |
| Cry1Ab15 | AAO13302 | Li et al | 2001 | Bt B-Hm-16 | |
| Cry1Ab16 | AAK55546 | Yu et al | 2002 | Bt AC-11 | |
| Cry1Ab17 | AAT46415 | Huang et al | 2004 | Bt WB9 | |
| Cry1Ab18 | AAQ88259 | Stobdan et al | 2004 | Bt | |
| Cry1Ab19 | AAW31761 | Zhong et al | 2005 | Bt X-2 | |
| Cry1Ab20 | ABB72460 | Liu et al | 2006 | BtC008 | |
| Cry1Ab21 | ABS18384 | Swiecicka et al | 2007 | Bt IS5056 | |
| Cry1Ab22 | ABW87320 | Wu and Feng | 2008 | BtS2491Ab | |
| Cry1Ab-like | AAK14336 | Nagarathinam et al | 2001 | Bt kunthala RX24 | uncertain sequence |
| Cry1Ab-like | AAK14337 | Nagarathinam et al | 2001 | Bt kunthala RX28 | uncertain sequence |
| Cry1Ab-like | AAK14338 | Nagarathinam et al | 2001 | Bt kunthala RX27 | uncertain sequence |
| Cry1Ab-like | ABG88858 | Lin et al | 2006 | Bt ly4a3 | insufficient sequence |
| Cry1Ac1 | AAA22331 | Adang et al | 1985 | Bt kurstaki HD73 | |
| Cry1Ac2 | AAA22338 | Von Tersch et al | 1991 | Bt kenyae | |
| Cry1Ac3 | CAA38098 | Dardenne et al | 1990 | Bt BTS89A | |
| Cry1Ac4 | AAA73077 | Feitelson | 1991 | Bt kurstaki PS85A1 | |
| Cry1Ac5 | AAA22339 | Feitelson | 1992 | Bt kurstaki PS81GG | |
| Cry1Ac6 | AAA86266 | Masson et al | 1994 | Bt kurstaki NRD-12 | |
| Cry1Ac7 | AAB46989 | Herrera et al | 1994 | Bt kurstaki HD73 | |
| Cry1Ac8 | AAC44841 | Omolo et al | 1997 | Bt kurstaki HD73 | |
| Cry1Ac9 | AAB49768 | Gleave et al | 1992 | Bt DSIR732 | |
| Cry1Ac10 | CAA05505 | Sun | 1997 | Bt kurstaki YBT-1520 | |
| Cry1Ac11 | CAA10270 | Makhdoom & Riazuddin | 1998 | | |
| Cry1Ac12 | I12418 | Ely & Tippett | 1995 | Bt A20 | DNA sequence only |
| Cry1Ac13 | AAD38701 | Qiao et al | 1999 | Bt kurstaki HD1 | |
| Cry1Ac14 | AAQ06607 | Yao et al | 2002 | Bt Ly30 | |
| Cry1Ac15 | AAN07788 | Tzeng et al | 2001 | Bt from Taiwan | |
| Cry1Ac16 | AAU87037 | Zhao et al | 2005 | Bt H3 | |
| Cry1Ac17 | AAX18704 | Hire et al | 2005 | Bt kenyae HD549 | |
| Cry1Ac18 | AAY88347 | Kaur & Allam | 2005 | Bt SK-729 | |
| Cry1Ac19 | ABD37053 | Gao et al | 2005 | Bt C-33 | |
| Cry1Ac20 | ABB89046 | Tan et al | 2005 | | |
| Cry1Ac21 | AAY66992 | Sauka et al | 2005 | INTA Mol-12 | |
| Cry1Ac22 | ABZ01836 | Zhang & Fang | 2008 | Bt W015-1 | |
| Cry1Ac23 | CAQ30431 | Kashyap et al | 2008 | Bt | |
| Cry1Ac24 | ABL01535 | Arango et al | 2008 | Bt 146-158-01 | |
| Cry1Ac25 | FJ513324 | Guan Peng et al | 2008 | Bt Tm37-6 | No NCBI link July 09 |
| Cry1Ac26 | FJ617446 | Guan Peng et al | 2009 | Bt Tm41-4 | No NCBI link July 09 |
| Cry1Ac27 | FJ617447 | Guan Peng et al | 2009 | Bt Tm44-1B | No NCBI link July 09 |
| Cry1Ac28 | ACM90319 | Li et al | 2009 | Bt Q-12 | |
| Cry1Ad1 | AAA22340 | Feitelson | 1993 | Bt aizawai PS81I | |

(continued)

| Name | Acc No. | Authors | Year | Source Strain | Comment |
|------|---------|---------|------|---------------|---------|
| Cry1Ad2 | CAA01880 | Anonymous | 1995 | Bt PS81RR1 | |
| Cry1Ae1 | AAA22410 | Lee & Aronson | 1991 | Bt alesti | |
| Cry1Af1 | AAB82749 | Kang et al | 1997 | Bt NT0423 | |
| Cry1Ae1 | AAD46137 | Mustafa | 1999 | | |
| Cry1Ah1 | AAQ14326 | Tan et al | 2000 | | |
| Cry1Ah2 | ABB76664 | Qi et al | 2005 | Bt alesti | |
| Cry1Ai1 | AAO39719 | Wang et al | 2002 | | |
| Cry1A-like | AAK14339 | Nagarathinam et al | 2001 | Bt kunthala nags3 | uncertain sequence |
| Cry1Ba1 | CAA29898 | Brizzard & Whiteley | 1988 | Bt thuringiensis HD2 | |
| Cry1Ba2 | CAA65003 | Soetaert | 1996 | Bt entomocidus HD110 | |
| Cry1Ba3 | AAK63251 | Zhang et al | 2001 | | |
| Cry1Ba4 | AAK51084 | Nathan et al | 2001 | Bt entomocidus HD9 | |
| Cry1Ba5 | ABO20894 | Song et al | 2007 | Bt sfw-12 | |
| Cry1Ba6 | ABL60921 | Martins et al | 2006 | Bt S601 | |
| Cry1Bb1 | AAA22344 | Donovan et al | 1994 | Bt EG5847 | |
| Cry1Bc1 | CAA86568 | Bishop et al | 1994 | Bt morrisoni | |
| Cry1Bd1 | AAD10292 | Kuo et al | 2000 | Bt wuhanensis HD525 | |
| Cry1Bd2 | AAM93496 | Isakova et al | 2002 | Bt 834 | |
| Cry1Be1 | AAC32850 | Payne et al | 1998 | Bt PS158C2 | |
| Cry1Be2 | AAQ52387 | Baum et al | 2003 | | |
| Cry1Be3 | FJ716102 | Xiaodong Sun et al | 2009 | Bt | No NCBI link July 09 |
| Cry1Bf1 | CAC50778 | Arnaut et al | 2001 | | |
| Cry1Bf2 | AAQ52380 | Baum et al | 2003 | | |
| Cry1Bg1 | AAO39720 | Wang et al | 2002 | | |
| Cry1Ca1 | CAA30396 | Honee et al | 1988 | Bt entomocidus 60.5 | |
| Cry1Ca2 | CAA31951 | Sanchis et al | 1989 | Bt aizawai 7.29 | |
| Cry1Ca3 | AAA22343 | Feitelson | 1993 | Bt aizawai PS81I | |
| Cry1Ca4 | CAA01886 | Van Mellaert et al | 1990 | Bt entomocidus HD110 | |
| Cry1Ca5 | CAA65457 | Strizhov | 1996 | Bt aizawai 7.29 | |
| Cry1Ca6 | AAF37224 | Yu et al | 2000 | Bt AF-2 | |
| Cry1Ca7 | AAG50438 | Aixing et al | 2000 | Bt J8 | |
| Cry1Ca8 | AAM00264 | Chen et al | 2001 | Bt c002 | |
| Cry1Ca9 | AAL79362 | Kao et al | 2003 | Bt G10-01A | |
| Cry1Ca10 | AAN16462 | Lin et al | 2003 | Bt E05-20a | |
| Cry1Ca11 | AAX53094 | Cai et al | 2005 | Bt C-33 | |
| Cry1Cb1 | M97880 | Kalman et al | 1993 | Bt galleriae HD29 | DNA sequence only |
| Cry1Cb2 | AAG35409 | Song et al | 2000 | Bt c001 | |
| Cry1Cb3 | ACD50894 | Huang et al | 2008 | Bt 087 | |
| Cry1Cb-like | AAX63901 | Thammasittirong et al | 2005 | Bt TA476-1 | insufficient sequence |
| Cry1Da1 | CAA38099 | Hofte et al | 1990 | Bt aizawai HD68 | |
| Cry1Da2 | I76415 | Payne & Sick | 1997 | | DNA sequence only |
| Cry1Db1 | CAA80234 | Lambert | 1993 | Bt BTS00349A | |
| Cry1Db2 | AAK48937 | Li et al | 2001 | Bt B-Pr-88 | |
| Cry1Dc1 | ABK35074 | Lertwiriyawong et al | 2006 | Bt JC291 | |
| Cry1Ea1 | CAA37933 | Visser et al | 1990 | Bt kenyae 4F1 | |
| Cry1Ea2 | CAA39609 | Bosse et al | 1990 | Bt kenyae | |
| Cry1Ea3 | AAA22345 | Feitelson | 1991 | Bt kenyae PS81F | |
| Cry1Ea4 | AAD04732 | Barboza-Corona et al | 1998 | Bt kenyae LBIT-147 | |
| Cry1Ea5 | A15535 | Botterman et al | 1994 | | DNA sequence only |
| Cry1Ea6 | AAL50330 | Sun et al | 1999 | Bt YBT-032 | |

(continued)

| Name | Acc No. | Authors | Year | Source Strain | Comment |
|------|---------|---------|------|---------------|---------|
| Cry1Ea7 | AAW72936 | Huehne et al | 2005 | Bt JC190 | |
| Cry1Ea8 | ABX11258 | Huang et al | 2007 | Bt HZM2 | |
| Cry1Eb1 | AAA22346 | Feitelson | 1993 | Bt aizawai PS81A2 | |
| Cry1Fa1 | AAA22348 | Chambers et al | 1991 | Bt aizawai EG6346 | |
| Cry1Fa2 | AAA22347 | Feitelson | 1993 | Bt aizawai PS81I | |
| Cry1Fb1 | CAA80235 | Lambert | 1993 | Bt BTS00349A | |
| Cry1Fb2 | BAA25298 | Masuda & Asano | 1998 | Bt morrisoni INA67 | |
| Cry1Fb3 | AAF21767 | Song et al | 1998 | Bt morrisoni | |
| Cry1Fb4 | AAC10641 | Payne et al | 1997 | | |
| Cry1Fb5 | AAO13295 | Li et al | 2001 | Bt B-Pr-88 | |
| Cry1Fb6 | ACD50892 | Huang et al | 2008 | Bt 012 | |
| Cry1Fb7 | ACD50893 | Huang et al | 2008 | Bt 087 | |
| Cry1Ga1 | CAA80233 | Lambert | 1993 | Bt BTS0349A | |
| Cry1Ga2 | CAA70506 | Shevelev et al | 1997 | Bt wuhanensis | |
| Cry1Gb1 | AAD10291 | Kuo & Chak | 1999 | Bt wuhanensis HD525 | |
| Cry1Gb2 | AAO13756 | Li et al | 2000 | Bt B-Pr-88 | |
| Cry1Gc | AAQ52381 | Baum et al | 2003 | | |
| Cry1Ha1 | CAA80236 | Lambert | 1993 | Bt BTS02069AA | |
| CrvlHbl | AAA79694 | Koo et al | 1995 | Bt morrisoni BF190 | |
| Cry1H- like | AAF01213 | Srifah et al | 1999 | Bt JC291 | insufficient sequence |
| Cry1Ia1 | CAA44633 | Tailor et al | 1992 | Bt kurstaki | |
| Cry1Ia2 | AAA22354 | Gleave et al | 1993 | Bt kurstaki | |
| Cry1Ia3 | AAC36999 | Shin et al | 1995 | Bt kurstaki HD1 | |
| Cry1Ia4 | AAB00958 | Kostichka et al | 1996 | Bt AB88 | |
| Cry1Ia5 | CAA70124 | Selvapandiyan | 1996 | Bt 61 | |
| Cry1Ia6 | AAC26910 | Zhong et al | 1998 | Bt kurstaki S101 | |
| Cry1Ia7 | AAM73516 | Porcar et al | 2000 | Bt | |
| Cry1Ia8 | AAK66742 | Song et al | 2001 | | |
| Cry1Ia9 | AAQ08616 | Yao et al | 2002 | Bt Ly30 | |
| Cry1Ia10 | AAP86782 | Espindola et al | 2003 | Bt thuringiensis | |
| Cry1Ia11 | CAC85964 | Tounsi et al | 2003 | Bt kurstaki BNS3 | |
| Cry1Ia12 | AAV53390 | Grossi de Sa et al | 2005 | Bt | |
| Cry1Ia13 | ABF83202 | Martins et al | 2006 | Bt | |
| Cry1Ia14 | ACG63871 | Liu & Guo | 2008 | Bt11 | |
| Cry1Ia15 | FJ617445 | Guan Peng et al | 2009 | Bt E-1B | No NCBI link July 2009 |
| Cry1Ia16 | FJ617448 | Guan Peng et al | 2009 | Bt E-1A | No NCBI link July 2009 |
| Cry1Ib1 | AAA82114 | Shin et al | 1995 | Bt entomocidus BP465 | |
| Cry1Ib2 | ABW88019 | Guan et al | 2007 | Bt PP61 | |
| Cry1Ib3 | ACD75515 | Liu & Guo | 2008 | Bt GS8 | |
| Cry1Ic1 | AAC62933 | Osman et al | 1998 | Bt C18 | |
| Cry1Ic2 | AAE71691 | Osman et al | 2001 | | |
| Cry1Id1 | AAD44366 | Choi | 2000 | | |
| Cry1Ie1 | AAG43526 | Song et al | 2000 | Bt BTC007 | |
| Cry1If1 | AAQ52382 | Baum et al | 2003 | | |
| Cry1I-like | AAC31094 | Payne et al | 1998 | | insufficient sequence |
| Cry1I-like | ABG88859 | Lin & Fang | 2006 | Bt ly4a3 | insufficient sequence |
| Cry1Ja1 | AAA22341 | Donovan | 1994 | Bt EG5847 | |
| Cry1Jb1 | AAA98959 | Von Tersch & Gonzalez | 1994 | Bt EG5092 | |

(continued)

| Name | Acc No. | Authors | Year | Source Strain | Comment |
|------|---------|---------|------|---------------|---------|
| Cry1Jc1 | AAC31092 | Payne et al | 1998 | | |
| Cry1Jc2 | AAQ52372 | Baum et al | 2003 | | |
| Cry1Jd1 | CAC50779 | Arnaut et al | 2001 | Bt | |
| Cry1Ka1 | AAB00376 | Koo et al | 1995 | Bt morrisoni BF190 | |
| Cry1La1 | AAS60191 | Je et al | 2004 | Bt kurstaki K1 | |
| Cry1-like | AAC31091 | Payne et al | 1998 | | insufficient sequence |
| Cry2Aa1 | AAA22335 | Donovan et al | 1989 | Bt kurstaki | |
| Cry2Aa2 | AAA83516 | Widner & Whiteley | 1989 | Bt kurstaki HD1 | |
| Cry2Aa3 | D86064 | Sasaki et al | 1997 | Bt sotto | DNA sequence only |
| Cry2Aa4 | AAC04867 | Misra et al | 1998 | Bt kenyae HD549 | |
| Cry2Aa5 | CAA10671 | Yu & Pang | 1999 | Bt SL39 | |
| Cry2Aa6 | CAA10672 | Yu & Pang | 1999 | Bt YZ71 | |
| Crv2Aa7 | CAA10670 | Yu & Pang | 1999 | Bt CY29 | |
| Cry2Aa8 | AAO13734 | Wei et al | 2000 | Bt Dongbei 66 | |
| Cry2Aa9 | AAO13750 | Zhang et al | 2000 | | |
| Cry2Aa10 | AAQ04263 | Yao et al | 2001 | | |
| Cry2Aa11 | AAQ52384 | Baum et al | 2003 | | |
| Cry2Aa12 | ABI83671 | Tan et al | 2006 | Bt Rpp39 | |
| Cry2Aa13 | ABL01536 | Arango et al | 2008 | Bt 146-158-01 | |
| Cry2Aa14 | ACF04939 | Hire et al | 2008 | Bt HD-550 | |
| Cry2Ab1 | AAA22342 | Widner & Whiteley | 1989 | Bt kurstaki HD1 | |
| Cry2Ab2 | CAA39075 | Dankocsik et al | 1990 | Bt kurstaki HD1 | |
| Cry2Ab3 | AAG36762 | Chen et al | 1999 | Bt BTC002 | |
| Cry2Ab4 | AAO13296 | Li et al | 2001 | Bt B-Pr-88 | |
| Cry2Ab5 | AAQ04609 | Yao et al | 2001 | Bt ly30 | |
| Cry2Ab6 | AAP59457 | Wang et al | 2003 | Bt WZ-7 | |
| Cry2Ab7 | AAZ66347 | Udayasuriyan et al | 2005 | Bt 14-1 | |
| Cry2Ab8 | ABC95996 | Huang et al | 2006 | Bt WB2 | |
| Cry2Ab9 | ABC74968 | Zhang et al | 2005 | Bt LLB6 | |
| Cry2Ab10 | EF157306 | Lin et al | 2006 | Bt LyD | |
| Cry2Ab11 | CAM84575 | Saleem et al | 2007 | Bt CMBL-BT1 | |
| Cry2Ab12 | ABM21764 | Lin et al | 2007 | Bt LyD | |
| Cry2Ab13 | ACG76120 | Zhu et al | 2008 | Bt ywc5-4 | |
| Cry2Ab14 | ACG76121 | Zhu et al | 2008 | Bt Bts | |
| Cry2Ac1 | CAA40536 | Aronson | 1991 | Bt shanghai S1 | |
| Cry2Ac2 | AAG35410 | Song et al | 2000 | | |
| Cry2Ac3 | AAQ52385 | Baum et al | 2003 | | |
| Cry2Ac4 | ABC95997 | Huang et al | 2006 | Bt WB9 | |
| Cry2Ac5 | ABC74969 | Zhang et al | 2005 | | |
| Cry2Ac6 | ABC74793 | Xia et al | 2006 | Bt wuhanensis | |
| Cry2Ac7 | CAL18690 | Saleem et al | 2008 | Bt SBSBT-1 | |
| Cry2Ac8 | CAM09325 | Saleem et al | 2007 | Bt CMBL-BT1 | |
| Cry2Ac9 | CAM09326 | Saleem et al | 2007 | Bt CMBL-BT2 | |
| Cry2Ac10 | ABN15104 | Bai et al | 2007 | Bt QCL-1 | |
| Cry2Ac11 | CAM83895 | Saleem et al | 2007 | Bt HD29 | |
| Cry2Ac12 | CAM83896 | Saleem et al | 2007 | Bt CMBL-BT3 | |
| Cry2Ad1 | AAF09583 | Choi et al | 1999 | Bt BR30 | |
| Cry2Ad2 | ABC86927 | Huang et al | 2006 | Bt WB10 | |
| Cry2Ad3 | CAK29504 | Saleem et al | 2006 | Bt 5_2AcT(1) | |
| Cry2Ad4 | CAM32331 | Saleem et al | 2007 | Bt CMBL-BT2 | |

(continued)

| Name | Acc No. | Authors | Year | Source Strain | Comment |
|------|---------|---------|------|---------------|---------|
| Cry2Ad5 | CAO78739 | Saleem et al | 2007 | Bt HD29 | |
| Cry2Ae1 | AAQ52362 | Baum et al | 2003 | | |
| Cry2Af1 | ABO30519 | Beard et al | 2007 | Bt C81 | |
| Cry2Ag | ACH91610 | Zhu et al | 2008 | Bt JF 19-2 | |
| Cry2Ah | EU939453 | Zhang et al | 2008 | Bt | No NCBI link July 09 |
| Cry2Ah2 | ACL80665 | Zhang et al | 2009 | Bt BRC-ZQL3 | |
| Cry2Ai | FJ788388 | Udayasuriyan et al | 2009 | Bt | No NCBI link July 09 |
| Cry3Aa1 | AAA22336 | Herrnstadt et al | 1987 | Bt san diego | |
| Cry3Aa2 | AAA22541 | Sekar et al | 1987 | Bt tenebrionis | |
| Cry3Aa3 | CAA68482 | Hofte et al | 1987 | | |
| Cry3Aa4 | AAA22542 | McPherson et al | 1988 | Bt tenebrionis | |
| Cry3Aa5 | AAA50255 | Donovan et al | 1988 | Bt morrisoni EG2158 | |
| Cry3Aa6 | AAC43266 | Adams et al | 1994 | Bt tenebrionis | |
| Cry3Aa7 | CAB41411 | Zhang et al | 1999 | Bt 22 | |
| Cry3Aa8 | AAS79487 | Gao and Cai | 2004 | Bt YM-03 | |
| Cry3Aa9 | AAW05659 | Bulla and Candas | 2004 | Bt UTD-00 1 | |
| Cry3Aa10 | AAU29411 | Chen et al | 2004 | Bt 886 | |
| Cry3Aa11 | AAW82872 | Kurt et al | 2005 | Bt tenebrionis Mm2 | |
| Cry3Aa12 | ABY49136 | Sezen et al | 2008 | Bt tenebrionis | |
| Cry3Ba1 | CAA34983 | Sick et al | 1990 | Bt tolworthi 43F | |
| Cry3Ba2 | CAA00645 | Peferoen et al | 1990 | Bt PGSI208 | |
| Cry3Bb1 | AAA22334 | Donovan et al | 1992 | Bt EG4961 | |
| Cry3Bb2 | AAA74198 | Donovan et al | 1995 | Bt EG5144 | |
| Cry3Bb3 | I15475 | Peferoen et al | 1995 | | DNA sequence only |
| Cry3Ca1 | CAA42469 | Lambert et al | 1992 | Bt kurstaki BtI109P | |
| Cry4Aa1 | CAA68485 | Ward & Ellar | 1987 | Bt israelensis | |
| Cry4Aa2 | BAA00179 | Sen et al | 1988 | Bt israelensis HD522 | |
| Cry4Aa3 | CAD30148 | Berry et al | 2002 | Bt israelensis | |
| Cry4A-like | AAY96321 | Mahalakshmi et al | 2005 | Bt LDC-9 | insufficient sequence |
| Cry4Ba1 | CAA30312 | Chungjatpornchai et al | 1988 | Bt israelensis 4Q2-72 | |
| Cry4Ba2 | CAA30114 | Tungpradubkul et al | 1988 | Bt israelensis | |
| Cry4Ba3 | AAA22337 | Yamamoto et al | 1988 | Bt israelensis | |
| Cry4Ba4 | BAA00178 | Sen et al | 1988 | Bt israelensis HD522 | |
| Cry4Ba5 | CAD30095 | Berry et al | 2002 | Bt israelensis | |
| Cry4Ba-like | ABC47686 | Mahalakshmi et al | 2005 | Bt LDC-9 | insufficient sequence |
| Cry4Ca1 | EU646202 | Shu et al | 2008 | | No NCBI link July 09 |
| Cry4Cb1 | FJ403208 | Jun & Furong | 2008 | Bt HS18-1 | No NCBI link July 09 |
| Cry4Cb2 | FJ597622 | Jun & Furong | 2008 | Bt Ywc2-8 | No NCBI link July 09 |
| Cry4Cc1 | FJ403207 | Jun & Furong | 2008 | Bt MC28 | No NCBI link July 09 |
| Cry5Aa1 | AAA67694 | Narva et al | 1994 | Bt darmstadiensis PS17 | |
| Cry5Ab1 | AAA67693 | Narva et al | 1991 | Bt darmstadiensis PS17 | |
| Cry5Ac1 | 134543 | Payne et al | 1997 | | DNA sequence only |
| Cry5Ad1 | ABQ82087 | Lenane et al | 2007 | Bt L366 | |
| Cry5Ba1 | AAA68598 | Foncerrada & Narva | 1997 | Bt PS86Q3 | |
| Cry5Ba2 | ABW88932 | Guo et al | 2008 | YBT 1518 | |
| Cry6Aa1 | AAA22357 | Narva et al | 1993 | Bt PS52A1 | |
| Cry6Aa2 | AAM46849 | Bai et al | 2001 | YBT 1518 | |
| Cry6Aa3 | ABH03377 | Jia et al | 2006 | Bt 96418 | |
| Cry6Ba1 | AAA22358 | Narva et al | 1991 | Bt PS69D1 | |
| Cry7Aa1 | AAA22351 | Lambert et al | 1992 | Bt galleriae PGSI245 | |

(continued)

| Name | Acc No. | Authors | Year | Source Strain | Comment |
|------|---------|---------|------|---------------|---------|
| Cry7Ab1 | AAA21120 | Narva & Fu | 1994 | Bt dakota HD511 | |
| Cry7Ab2 | AAA21121 | Narva & Fu | 1994 | Bt kumamotoensis 867 | |
| Cry7Ab3 | ABX24522 | Song et al | 2008 | Bt WZ-9 | |
| Cry7Ab4 | EU380678 | Shu et al | 2008 | Bt | No NCBI link July 09 |
| Cry7Ab5 | ABX79555 | Aguirre-Arzola et al | 2008 | Bt monterrey GM-33 | |
| Cry7Ab6 | ACI44005 | Deng et al | 2008 | Bt HQ122 | |
| Cry7Ab7 | FJ940776 | Wang et al | 2009 | | No NCBI link Sept 09 |
| Cry7Ab8 | GU145299 | Feng Jing | 2009 | | No NCBI link Nov 09 |
| Cry7Ba1 | ABB70817 | Zhang et al | 2006 | Bt huazhongensis | |
| Cry7Ca1 | ABR67863 | Gao et al | 2007 | Bt BTH-13 | |
| Cry7Da1 | ACQ99547 | Yi et al | 2009 | Bt LH-2 | |
| Cry8Aa1 | AAA21117 | Narva & Fu | 1992 | Bt kumamotoensis | |
| Cry8Ab1 | EU044830 | Cheng et al | 2007 | Bt B-JJX | No NCBI link July 09 |
| Cry8Ba1 | AAA21118 | Narva & Fu | 1993 | Bt kumamotoensis | |
| Cry8Bb1 | CAD57542 | Abad et al | 2002 | | |
| Cry8Bc1 | CAD57543 | Abad et al | 2002 | | |
| Cry8Ca1 | AAA21119 | Sato et al. | 1995 | Bt japonensis Buibui | |
| Cry8Ca2 | AAR98783 | Shu et al | 2004 | Bt HBF-1 | |
| Cry8Ca3 | EU625349 | Du et al | 2008 | Bt FTL-23 | No NCBI link July 09 |
| Cry8Da1 | BAC07226 | Asano et al | 2002 | Bt galleriae | |
| Cry8Da2 | BD133574 | Asano et al | 2002 | Bt | DNA sequence only |
| Cry8Da3 | BD133575 | Asano et al | 2002 | Bt | DNA sequence only |
| Cry8Db1 | BAF93483 | Yamaguchi et al | 2007 | Bt BBT2-5 | |
| Cry8Ea1 | AAQ73470 | Fuping et al | 2003 | Bt 185 | |
| Cry8Ea2 | EU047597 | Liu et al | 2007 | Bt B-DLL | No NCBI link July 09 |
| Cry8Fa1 | AAT48690 | Shu et al | 2004 | Bt 185 | also AAW81032 |
| Cry8Ga1 | AAT46073 | Shu et al | 2004 | Bt HBF-18 | |
| Cry8Ga2 | ABC42043 | Yan et al | 2008 | Bt 145 | |
| Cry8Ga3 | FJ198072 | Xiaodong et al | 2008 | Bt FCD114 | No NCBI link July 09 |
| Cry8Ha1 | EF465532 | Fuping et al | 2006 | Bt 185 | No NCBI link July 09 |
| Cry8Ia1 | EU381044 | Yan et al | 2008 | Bt su4 | No NCBI link July 09 |
| Cry8Ja1 | EU625348 | Du et al | 2008 | Bt FPT-2 | No NCBI link July 09 |
| Cry8Ka1 | FJ422558 | Quezado et al | 2008 | | No NCBI link July 09 |
| Cry8Ka2 | ACN87262 | Noguera & Ibarra | 2009 | Bt kenyae | |
| Cry8-like | FJ770571 | Noguera & Ibarra | 2009 | Bt canadensis | DNA sequence only |
| Cry8-like | ABS53003 | Mangena et al | 2007 | Bt | |
| Cry9Aa1 | CAA41122 | Shevelev et al | 1991 | Bt galleriae | |
| Cry9Aa2 | CAA41425 | Gleave et al | 1992 | Bt DSIR517 | |
| Cry9Aa3 | GQ249293 | Su et al | 2009 | Bt SC5(D2) | No NCBI link July 09 |
| Cry9Aa4 | GQ249294 | Su et al | 2009 | Bt T03C001 | No NCBI link July 09 |
| Cry9Aa like | AAQ52376 | Baum et al | 2003 | | incomplete sequence |
| Cry9Ba1 | CAA52927 | Shevelev et al | 1993 | Bt galleriae | |
| Cry9Bb1 | AAV28716 | Silva-Werneck et al | 2004 | Bt japonensis | |
| Cry9Ca1 | CAA85764 | Lambert et al | 1996 | Bt tolworthi | |
| Cry9Ca2 | AAQ52375 | Baum et al | 2003 | | |
| Cry9Da1 | BAA19948 | Asano | 1997 | Bt japonensis N141 | |
| Cry9Da2 | AAB97923 | Wasano & Ohba | 1998 | Bt japonensis | |
| Cry9Da3 | GQ249295 | Su et al | 2009 | Bt T03B001 | No NCBI link July 09 |
| Cry9Da4 | GQ249297 | Su et al | 2009 | Bt T03B001 | No NCBI link July 09 |

(continued)

| Name | Acc No. | Authors | Year | Source Strain | Comment |
|------|---------|---------|------|---------------|---------|
| Cry9Db1 | AAX78439 | Flannagan & Abad | 2005 | Bt kurstaki DP 1019 | |
| Cry9Ea1 | BAA34908 | Midoh & Oyama | 1998 | Bt aizawai SSK-10 | |
| Cry9Ea2 | AAO12908 | Li et al | 2001 | Bt B-Hm-16 | |
| Cry9Ea3 | ABM21765 | Lin et al | 2006 | Bt lyA | |
| Cry9Ea4 | ACE88267 | Zhu et al | 2008 | Bt ywc5-4 | |
| Cry9Ea5 | ACF04743 | Zhu et al | 2008 | Bts | |
| Cry9Ea6 | ACG63872 | Liu & Guo | 2008 | Bt 11 | |
| Cry9Ea7 | FJ380927 | Sun et al | 2008 | | No NCBI link July 09 |
| Cry9Ea8 | GQ249292 | Su et al | 2009 | GQ249292 | No NCBI link July 09 |
| Cry9Eb1 | CAC50780 | Arnaut et al | 2001 | | |
| Cry9Eb2 | GQ249298 | Su et al | 2009 | Bt T03B001 | No NCBI link July 09 |
| Cry9Ec1 | AAC63366 | Wasano et al | 2003 | Bt galleriae | |
| Cry9Ed1 | AAX78440 | Flannagan & Abad | 2005 | Bt kurstaki DP 1019 | |
| Cry9Ee1 | GQ249296 | Su et al | 2009 | Bt T03B001 | No NCBI link Aug 09 |
| Cry9-like | AAC63366 | Wasano et al | 1998 | Bt galleriae | insufficient sequence |
| Cry10Aa1 | AAA22614 | Thorne et al | 1986 | Bt israelensis | |
| Cry10Aa2 | E00614 | Aran & Toomasu | 1996 | Bt israelensis ONR-60A | DNA sequence only |
| Cry10Aa3 | CAD30098 | Berry et al | 2002 | Bt israelensis | |
| Cry10A like | DQ167578 | Mahalakshmi et al | 2006 | Bt LDC-9 | incomplete sequence |
| Cry11Aa1 | AAA22352 | Donovan et al | 1988 | Bt israelensis | |
| Cry11Aa2 | AAA22611 | Adams et al | 1989 | Bt israelensis | |
| Cry11Aa3 | CAD30081 | Berry et al | 2002 | Bt israelensis | |
| Cry11Aa-like | DQ166531 | Mahalakshmi et al | 2007 | Bt LDC-9 | incomplete sequence |
| Cry11Ba1 | CAA60504 | Delecluse et al | 1995 | Bt jegathesan 367 | |
| Cry11Bb1 | AAC97162 | Orduz et al | 1998 | Bt medellin | |
| Cry12Aa1 | AAA22355 | Narva et al | 1991 | BtPS33F2 | |
| Cry13Aa1 | AAA22356 | Narva et al | 1992 | Bt PS63B | |
| Cry14Aa1 | AAA21516 | Narva et al | 1994 | Bt sotto PS80JJ1 | |
| Cry15Aa1 | AAA22333 | Brown & Whiteley | 1992 | Bt thompsoni | |
| Cry16Aa1 | CAA63860 | Barloy et al | 1996 | Cb malaysia CH18 | |
| Cry17Aa1 | CAA67841 | Barloy et al | 1998 | Cb malaysia CH18 | |
| Cry18Aa1 | CAA67506 | Zhang et al | 1997 | Paenibacillus popilliae | |
| Cry18Ba1 | AAF89667 | Patel et al | 1999 | Paenibacillus popilliae | |
| Cry18Ca1 | AAF89668 | Patel et al | 1999 | Paenibacillus popilliae | |
| Cry19Aa1 | CAA68875 | Rosso & Delecluse | 1996 | Bt jegathesan 367 | |
| Cry19Ba1 | BAA32397 | Hwang et al | 1998 | Bt higo | |
| Cry20Aa1 | AAB93476 | Lee & Gill | 1997 | Bt fukuokaensis | |
| Cry20Ba1 | ACS93601 | Noguera & Ibarra | 2009 | Bt higo LBIT-976 | |
| Cry20-like | GQ144333 | Yi et al | 2009 | Bt Y-5 | DNA sequence only |
| Cry21Aa1 | I32932 | Payne et al | 1996 | | DNA sequence only |
| Cry21Aa2 | I66477 | Feitelson | 1997 | | DNA sequence only |
| Cry21Ba1 | BAC06484 | Sato & Asano | 2002 | Bt roskildiensis | |
| Cry22Aa1 | 134547 | Payne et al | 1997 | | DNA sequence only |
| Cry22Aa2 | CAD43579 | Isaac et al | 2002 | Bt | |
| Cry22Aa3 | ACD93211 | Du et al | 2008 | Bt FZ-4 | |
| Cry22Ab1 | AAK50456 | Baum et al | 2000 | Bt EG4140 | |
| Cry22Ab2 | CAD43577 | Isaac et al | 2002 | Bt | |
| Cry22Ba1 | CAD43578 | Isaac et al | 2002 | Bt | |

(continued)

| Name | Acc No. | Authors | Year | Source Strain | Comment |
|------|---------|---------|------|---------------|---------|
| Cry23Aa1 | AAF76375 | Donovan et al | 2000 | Bt | Binary with Cry37Aa1 |
| Cry24Aa1 | AAC61891 | Kawalek and Gill | 1998 | Bt jegathesan | |
| Cry24Ba1 | BAD32657 | Ohgushi et al | 2004 | Bt sotto | |
| Cry24Ca1 | CAJ43600 | Beron & Salerno | 2005 | Bt FCC-41 | |
| Cry25Aa1 | AAC61892 | Kawalek and Gill | 1998 | Bt jegathesan | |
| Cry26Aa1 | AAD25075 | Wojciechowska et al | 1999 | Bt finitimus B-1166 | |
| Cry27Aa1 | BAA82796 | Saitoh | 1999 | Bt higo | |
| Cry28Aa1 | AAD24189 | Wojciechowska et al | 1999 | Bt finitimus B-1161 | |
| Cry28Aa2 | AAG00235 | Moore and Debro | 2000 | Bt finitimus | |
| Cry29Aa1 | CAC80985 | Delecluse et al | 2000 | Bt medellin | |
| Cry30Aa1 | CAC80986 | Delecluse et al | 2000 | Bt medellin | |
| Cry30Ba1 | BAD00052 | Ito et al | 2003 | Bt entomocidus | |
| Cry30Ca1 | BAD67157 | Ohgushi et al | 2004 | Bt sotto | |
| Cry30Ca2 | ACU24781 | Sun and Park | 2009 | Bt jegathesan 367 | |
| Cry30Da1 | EF095955 | Shu et al | 2006 | Bt Y41 | No NCBI link July09 |
| Cry30Db1 | BAE80088 | Kishida et al | 2006 | aizawai BUN1-14 | |
| Cry30Ea1 | ACC95445 | Fang et al | 2007 | Bt S2160-1 | |
| Cry30Ea2 | FJ499389 | Jun et al | 2008 | Bt Ywc2-8 | No NCBI link July09 |
| Cry30Fa1 | ACI22625 | Tan et al | 2008 | Bt MC28 | |
| Cry30Ga1 | ACG60020 | Zhu et al | 2008 | Bt HS18-1 | |
| Cry31Aa1 | BAB11757 | Saitoh & Mizuki | 2000 | Bt 84-HS-1-11 | |
| Cry31Aa2 | AAL87458 | Jung and Cote | 2000 | Bt M15 | |
| Cry31Aa3 | BAE79808 | Uemori et al | 2006 | Bt B0195 | |
| Cry31Aa4 | BAF32571 | Yasutake et al | 2006 | Bt 79-25 | |
| Cry31Aa5 | BAF32572 | Yasutake et al | 2006 | Bt 92-10 | |
| Cry31Ab1 | BAE79809 | Uemori et al | 2006 | Bt B0195 | |
| Cry31Ab2 | BAF32570 | Yasutake et al | 2006 | Bt 31-5 | |
| Cry31Ac1 | BAF34368 | Yasutake et al | 2006 | Bt 87-29 | |
| Cry32Aa1 | AAG36711 | Balasubramanian al et | 2001 | Bt yunnanensis | |
| Cry32Ba1 | BAB78601 | Takebe et al | 2001 | Bt | |
| Cry32Ca1 | BAB78602 | Takebe et al | 2001 | Bt | |
| Cry32Da1 | BAB78603 | Takebe et al | 2001 | Bt | |
| Cry33Aa1 | AAL26871 | Kim et al | 2001 | Bt dakota | |
| Cry34Aa1 | AAG50341 | Ellis et al | 2001 | Bt PS80JJ1 | Binary with Cry35Aa1 |
| Cry34Aa2 | AAK64560 | Rupar et al | 2001 | Bt EG5899 | Binary with Cry35Aa2 |
| Cry34Aa3 | AAT29032 | Schnepf et al | 2004 | Bt PS69Q | Binary with Cry35Aa3 |
| Cry34Aa4 | AAT29030 | Schnepf et al | 2004 | Bt PS185GG | Binary with Cry35Aa4 |
| Cry34Ab1 | AAG41671 | Moellenbeck et al | 2001 | Bt PS149B1 | Binary with Cry35Ab1 |
| Cry34Ac1 | AAG50118 | Ellis et al | 2001 | BtPS167H2 | Binary with Cry35Ac1 |
| Cry34Ac2 | AAK64562 | Rupar et al | 2001 | Bt EG9444 | Binary with Cry35Ab2 |
| Cry34Ac3 | AAT29029 | Schnepf et al | 2004 | Bt KR1369 | Binary with Cry35Ab3 |

(continued)

| Name | Acc No. | Authors | Year | Source Strain | Comment |
|------|---------|---------|------|---------------|---------|
| Cry34Ba1 | AAK64565 | Rupar et al | 2001 | Bt EG4851 | Binary with Cry35Ba1 |
| Cry34Ba2 | AAT29033 | Schnepf et al | 2004 | Bt PS201L3 | Binary with Cry35Ba2 |
| Cry34Ba3 | AAT29031 | Schnepf et al | 2004 | Bt PS201HH2 | Binary with Cry35Ba3 |
| Cry35Aa1 | AAG50342 | Ellis et al | 2001 | Bt PS80JJ1 | Binary with Cry34Aa1 |
| Cry35Aa2 | AAK64561 | Rupar et al | 2001 | Bt EG5899 | Binary with Cry34Aa2 |
| Cry35Aa3 | AAT29028 | Schnepf et al | 2004 | Bt PS69Q | Binary with Cry34Aa3 |
| Cry35Aa4 | AAT29025 | Schnepf et al | 2004 | Bt PS185GG | Binary with Cry34Aa4 |
| Cry35Ab1 | AAG41672 | Moellenbeck et al | 2001 | Bt PS149B1 | Binary with Cry34Ab1 |
| Cry35Ab2 | AAK64563 | Rupar et al | 2001 | Bt EG9444 | Binary with Cry34Ac2 |
| Cry35Ab3 | AY536891 | AAT29024 | 2004 | Bt KR1369 | Binary with Cry34Ab3 |
| Cry35Ac1 | AAG50117 | Ellis et al | 2001 | Bt PS167H2 | Binary with Cry34Ac1 |
| Cry35Ba1 | AAK64566 | Rupar et al | 2001 | Bt EG4851 | Binary with Cry34Ba1 |
| Cry35Ba2 | AAT29027 | Schnepf et al | 2004 | Bt PS201L3 | Binary with Cry34Ba2 |
| Cry35Ba3 | AAT29026 | Schnepf et al | 2004 | Bt PS201HH2 | Binary with Cry34Ba3 |
| Cry36Aa1 | AAK64558 | Rupar et al | 2001 | Bt | |
| Cry37Aa1 | AAF76376 | Donovan et al | 2000 | Bt | Binary with Cry23Aa |
| Cry38Aa1 | AAK64559 | Rupar et al | 2000 | Bt | |
| Cry39Aa1 | BAB72016 | Ito et al | 2001 | Bt aizawai | |
| Cry40Aa1 | BAB72018 | Ito et al | 2001 | Bt aizawai | |
| Cry40Ba1 | BAC77648 | Ito et al | 2003 | Bun1-14 | |
| Cry40Ca1 | EU381045 | Shu et al | 2008 | Bt Y41 | No NCBI link July09 |
| Cry40Da1 | ACF15199 | Zhang et al | 2008 | Bt S2096-2 | |
| Cry41Aa1 | BAD35157 | Yamashita et al | 2003 | Bt A1462 | |
| Cry41Ab1 | BAD35163 | Yamashita et al | 2003 | Bt A1462 | |
| Cry42Aa1 | BAD35166 | Yamashita et al | 2003 | Bt A1462 | |
| Cry43Aa1 | BAD15301 | Yokoyama and Tanaka | 2003 | P. lentimorbus semadara | |
| Cry43Aa2 | BAD95474 | Nozawa | 2004 | P. popilliae popilliae | |
| Cry43Ba1 | BAD15303 | Yokoyama and Tanaka | 2003 | P. lentimorbus semadara | |
| Cry43-like | BAD15305 | Yokoyama and Tanaka | 2003 | P. lentimorbus semadara | |
| Cry44Aa | BAD08532 | Ito et al | 2004 | Bt entomocidus INA288 | |
| Cry45Aa | BAD22577 | Okumura et al | 2004 | Bt 89-T-34-22 | |
| Cry46Aa | BAC79010 | Ito et al | 2004 | Bt dakota | |
| Cry46Aa2 | BAG68906 | Ishikawa et al | 2008 | Bt A1470 | |
| Cry46Ab | BAD35170 | Yamagiwa et al | 2004 | Bt | |

24

(continued)

| Name | Acc No. | Authors | Year | Source Strain | Comment |
|---|---|---|---|---|---|
| Cry47Aa | AAY24695 | Kongsuwan et al | 2005 | Bt CAA890 | |
| Cry48Aa | CAJ18351 | Jones and Berry | 2005 | Bs IAB59 | binary with 49Aa |
| Cry48Aa2 | CAJ86545 | Jones and Berry | 2006 | Bs 47-6B | binary with 49Aa2 |
| Cry48Aa3 | CAJ86546 | Jones and Berry | 2006 | Bs NHA15b | binary with 49Aa3 |
| Cry48Ab | CAJ86548 | Jones and Berry | 2006 | Bs LP1G | binary with 49Ab1 |
| Cry48Ab2 | CAJ86549 | Jones and Berry | 2006 | Bs 2173 | binary with 49Aa4 |
| Cry49Aa | CAH56541 | Jones and Berry | 2005 | Bs IAB59 | binary with 48Aa |
| Cry49Aa2 | CAJ86541 | Jones and Berry | 2006 | Bs 47-6B | binary with 48Aa2 |
| Cry49Aa3 | CAJ86543 | Jones and Berry | 2006 | BsNHA15b | binary with 48Aa3 |
| Cry49Aa4 | CAJ86544 | Jones and Berry | 2006 | Bs 2173 | binary with 48Ab2 |
| Cry49Ab1 | CAJ86542 | Jones and Berry | 2006 | Bs LP1G | binary with 48Ab1 |
| Cry50Aa1 | BAE86999 | Ohgushi et al | 2006 | Bt sotto | |
| Cry51Aa1 | ABI14444 | Meng et al | 2006 | Bt F14-1 | |
| Cry52Aa1 | EF613489 | Song et al | 2007 | Bt Y41 | No NCBI link July09 |
| Cry52Ba1 | FJ361760 | Jun et al | 2008 | Bt BM59-2 | No NCBI link July09 |
| Cry53Aa1 | EF633476 | Song et al | 2007 | Bt Y41 | No NCBI link July09 |
| Cry53Ab1 | FJ361759 | Jun et al | 2008 | Bt MC28 | No NCBI link July09 |
| Cry54Aa1 | ACA52194 | Tan et al | 2009 | Bt MC28 | |
| Cry55Aa1 | ABW88931 | Guo et al | 2008 | YBT 1518 | |
| Cry55Aa2 | AAE33526 | Bradfisch et al | 2000 | BT Y41 | |
| Cry56Aa1 | FJ597621 | Jun & Furong | 2008 | Bt Ywc2-8 | No NCBI link July09 |
| Cry56Aa2 | GQ483512 | Guan Peng et al | 2009 | Bt G7-1 | No NCBI link Aug09 |
| Cry57Aa1 | ANC87261 | Noguera & Ibarra | 2009 | Bt kim | |
| Cry58Aa1 | ANC87260 | Noguera & Ibarra | 2009 | Bt entomocidus | |
| Cry59Aa1 | ACR43758 | Noguera & Ibarra | 2009 | Bt kim LBIT-980 | |

| **Vip3Aa1** | Vip3Aa | AAC37036 | Estruch et al | 1996 | PNAS 93, 5389-5394 | AB88 | |
|---|---|---|---|---|---|---|---|
| Vip3Aa2 | Vip3Ab | AAC37037 | Estruch et al | 1996 | PNAS 93, 5389-5394 | AB424 | |
| Vip3Aa3 | Vip3Ac | | Estruch et al | 2000 | US 6137033 Oct 2000 | | |
| Vip3Aa4 | PS36A Sup | AAR81079 | Feitelson et al | 1998 | US 6656908 Dec 2003 | Bt PS36A | WO9818932 (A2,A3) 7 May 1998 |
| Vip3Aa5 | PS81F Sup | AAR81080 | Feitelson et al | 1998 | US 6656908 Dec 2003 | Bt PS81F | WO9818932 (A2,A3) 7 May 1998 |
| Vip3Aa6 | Jav90 Sup | AAR81081 | Feitelson et al | 1998 | US 6656908 Dec 2003 | Bt | WO9818932 (A2,A3) 7 May 1998 |
| Vip3Aa7 | Vip83 | AAK95326 | Cai et al | 2001 | unpublished | Bt YBT-833 | |
| Vip3Aa8 | Vip3A | AAK97481 | Loguercio et al | 2001 | unpublished | Bt HD125 | |
| Vip3Aa9 | VipS | CAA76665 | | 2001 | Selvapandiyan et al | Bt A13 | |

(continued)

| Vip3Aa10 | Vip3V | AAN60738 | Doss et al | 2002 | Protein Expr Purif. 26. 82- 88 | Bt | |
| Vip3Aa11 | Vip3A | AAR36859 | Liu et al | 2003 | unpublished | Bt C9 | |
| Vip3Aa12 | Vip3A-WB5 | AAM22456 | Wu and Guan | 2003 | unpublished | Bt | |
| Vip3Aa13 | Vip3A | AAL69542 | Chen et al | 2002 | Sheng Wu Gong Cheng Xue Bao 18, 687-692 | Bt S184 | |
| Vip3Aa14 | Vip | AAQ12340 | Polumetla et al | 2003 | unpublished | Bt tolworthi | |
| Vip3Aa15 | Vip3A | AAP51131 | Wu et | 2004 | | BtWB50Bt WB50 | |
| Vip3Aa16 | Vip3LB | AAW65132 | Mesrati et al | 2005 | FEMS Micro Lett 244, 353-358 | Bt | |
| Vip3Aa17 | Jav90 | | Feitelson et al | 1999 | US 6603063 Aug 2003 | Javelin 1990 | WO9957282 (A2,A3) 11Nov 1999 |
| Vip3Aa18 | | AAX49395 | Cai and Xiao | 2005 | unpublished | Bt 9816C | |
| Vip3Aa19 | Vip3ALD | DQ241674 | Liu et al | 2006 | unpublished | Bt AL | |
| Vip3Aa19 | Vip3A-1 | DQ539887 | Hart et al | 2006 | unpublished | | |
| Vip3Aa20 | Vip3A-2 | DQ539888 | Hart et al | 2006 | unpublished | | |
| Vip3Aa21 | Vip | ABD84410 | Panbangred | 2006 | unpublished | Bt aizawai | |
| Vip3Aa22 | Vip3A-LS1 | AAY41427 | Lu et al | 2005 | unpublished | Bt LS1 | |
| Vip3Aa23 | Vip3A-LS8 | AAY41428 | Lu et al | 2005 | unpublished | Bt LS8 | |
| Vip3Aa24 | | BI 880913 | Song et al | 2007 | unpublished | Bt WZ-7 | |
| Vip3Aa25 | | EF608501 | Hsieh et al | 2007 | unpublished | | |
| Vip3Aa26 | | EU294496 | Shen and Guo | 2007 | unpublished | Bt TF9 | |
| Vip3Aa27 | | EU332167 | Shen and Guo | 2007 | unpublished | Bt 16 | |
| Vip3Aa28 | | FJ494817 | Xiumei Yu | 2008 | unpublished | Bt JF23-8 | |
| Vip3Aa29 | | FJ626674 | Xieumei et al | 2009 | unpublished | Bt JF21-1 Bt JF21-1 | |
| Vip3Aa30 | | FJ626675 | Xieumei et al | 2009 | unpublished | MD2-1 | |
| Vip3Aa31 | | FJ626676 | Xieumei et al | 2009 | unpublished | JF21-1 | |
| Vip3Aa32 | | FJ626677 | Xieumei et al | 2009 | unpublished | MD2-1 | |
| . | | | | | | | |
| **Vip3Ab1** | Vip3B | AAR40284 | Feitelson et al | 1999 | US 6603063 Aug 2003 | Bt KB59A4-6 | WO9957282 (A2,A3) 11Nov 1999 |
| Vip3Ab2 | Vip3D | AAY88247 | Feng and Shen | 2006 | unpublished | Bt | |

(continued)

| | | | | | | | |
|---|---|---|---|---|---|---|---|
| . | | | | | | | |
| **Vip3Ac1** | PS49C | | Narva et al | | US application 2004012871 6 | | |
| . | | | | | | | |
| **Vip3Ad1** | PS158C2 | | Narva et al | | US application 2004012871 6 | | |
| Vip3Ad2 | ISP3B | CAI43276 | Van Rie et al | 2005 | unpublished | Bt | |
| | | | | | | | |
| **Vip3Ae1** | ISP3C | CAI43277 | Van Rie et al | | unpublished | Bt | |
| | | | | | | | |
| **Vip3Af1** | ISP3A | CAI43275 | Van Rie et al | 2005 | unpublished | Bt | |
| Vip3Af2 | Vip3C | ADN08753 | Syngenta | | WO 03/075655 | | |
| | | | | | | | |
| **Vip3Ag1** | Vip3B | ADN08758 | Syngenta | | WO 02/078437 | | |
| Vip3Ag2 | | FJ556803 | Audtho et al | 2008 | | Bt | |
| | | | | | | | |
| **Vip3Ah1** | Vip3S | DQ832323 | Li and Shen | 2006 | unpublished | Bt | |
| | | | | | | | |
| **Vip3Ba1** | | AAV70653 | Rang et al | 2004 | unpublished | | |
| | | | | | | | |
| **Vip3Bb1** | Vip3Z | ADN08760 | Syngenta | | WO03/075655 | | |
| Vip3Bb2 | | EF439819 | Akhurst et al | 2007 | | | |

**Claims**

1. A transgenic plant comprising and expressing DNA encoding a Vip3Ab insecticidal protein and DNA encoding a Cry1F insecticidal protein, further comprising and expressing DNA encoding a third insecticidal protein, said third protein being selected from the group consisting of Cry1C, Cry1D, and Cry1Be.

2. Seed of a transgenic plant comprising and expressing the DNA encoding a Vip3Ab insecticidal protein and the DNA encoding a Cry1F protein, further comprising and expressing DNA encoding a third insecticidal protein, said third protein being selected from the group consisting of Cry1C, Cry1D, and Cry1Be.

3. A mixture of seeds comprising refuge seeds from non-Bt refuge plants, and a plurality of seeds of claim 2, wherein said refuge seeds comprise less than 40% of all the seeds in the mixture.

4. A composition for controlling lepidopteran pests comprising a host cell transformed to express effective amounts of a Cry1F core toxin-containing protein and a Vip3Ab protein, further comprising a third insecticidal protein, wherein third protein being selected from the group consisting of Cry1C, Cry1D, and Cry1Be, wherein said Cry1F core toxin-containing protein contains the N-terminal, insecticidally active, toxin portion of Cry1F.

5. The transgenic plant of claim 1 wherein said plant produces a fourth protein and a fifth protein selected from the group consisting of Cry2A, Cry1I, Cry1Ab, and DIG-3.

6. The transgenic plant of claim 1 wherein said plant produces a fourth protein selected from the group consisting of Cry2A, Cry1I, Cry1Ab, and DIG-3.

7. The transgenic plant of claim 6 wherein said third protein is a Cry1Be protein.

8. A mixture of seeds comprising refuge seeds from non-Bt refuge plants, and a plurality of seeds from a plant of claim 7 comprising and expressing the DNA encoding a Vip3Ab insecticidal protein and the DNA encoding a Cry1F protein, further comprising and expressing DNA encoding a Cry1Be insecticidal protein, and producing a fourth protein selected from the group consisting of Cry2A, Cry1I, Cry1Ab, and DIG-3, wherein said refuge seeds comprise less than 10% of all the seeds in the mixture.

9. A plant of any of claims 1 and 5 to 7 wherein said plant is selected from the group consisting of corn, soybeans, and cotton.

10. A plant cell of a plant of any of claims 1 and 5 to 7, wherein said plant cell comprises and expresses said DNA encoding said Cry1F insecticidal protein and said DNA encoding said Vip3Ab insecticidal protein, wherein said Cry1F insecticidal protein is at least 99% identical with SEQ ID NO: 1, and said Vip3Ab insecticidal protein is at least 99% identical with SEQ ID NO: 2.

11. A plant of any of claims 1 and 5 to 7, wherein said Cry1F insecticidal protein comprises SEQ ID NO: 1, and said Vip3Ab insecticidal protein comprises SEQ ID NO: 2.

**Patentansprüche**

1. Transgene Pflanze, umfassend und exprimierend eine DNA, die ein insektizides Vip3Ab-Protein kodiert, und eine DNA, die ein insektizides Cry1F-Protein kodiert, ferner umfassend und exprimierend eine DNA, die ein drittes insektizides Protein kodiert, wobei das dritte Protein ausgewählt ist aus der Gruppe bestehend aus Cry1C, Cry1D und Cry1Be.

2. Samen einer transgenen Pflanze, umfassend und exprimierend eine DNA, die ein insektizides Vip3Ab-Protein kodiert, und eine DNA, die ein insektizides Cry1F-Protein kodiert, ferner umfassend und exprimierend eine DNA, die ein drittes insektizides Protein kodiert, wobei das dritte Protein ausgewählt ist aus der Gruppe bestehend aus Cry1C, Cry1D, und Cry1Be.

3. Samenmischung, die Refugiumssamen von Nicht-Bt-Refugiumspflanzen und eine Vielzahl von Samen nach Anspruch 2 umfasst, wobei die Refugiumsmsamen weniger als 40 % aller Samen in der Mischung umfassen.

**4.** Zusammensetzung zum Bekämpfen von Lepidoptera-Schädlingen, umfassend eine Wirtszelle, die transformiert ist, um wirksame Mengen des Cry1F-Kerntoxinenthaltenden Proteins und eines Vip3Ab-Proteins zu exprimieren, ferner umfassend ein drittes insektizides Protein, wobei das dritte Protein ausgewählt ist aus der Gruppe bestehend aus Cry1C, Cry1D, und Cry1Be, wobei das Cry1F-Kerntoxin-enthaltende Protein den N-terminalen, insektizid wirksamen Toxinabschnitt von CrylF enthält.

**5.** Transgene Pflanze nach Anspruch 1, wobei die Pflanze ein viertes Protein und ein fünftes Protein erzeugt, ausgewählt aus der Gruppe bestehend aus Cry2A, Cry1I, Cry1Ab und DIG-3.

**6.** Transgene Pflanze nach Anspruch 1, wobei die Pflanze ein viertes Protein erzeugt, ausgewählt aus der Gruppe bestehend aus Cry2A, Cry1I, Cry1Ab und DIG-3.

**7.** Transgene Pflanze nach Anspruch 6, wobei das dritte Protein ein Cry1Be-Protein ist.

**8.** Samenmischung, die Refugiumssamen von Nicht-Bt-Refugiumspflanzen und eine Vielzahl von Samen aus einer Pflanze nach Anspruch 7 umfasst, umfassend und exprimierend eine DNA, die ein insektizides Vip3Ab-Protein kodiert, und eine DNA, die ein Cry1F-Protein kodiert, ferner umfassend und exprimierend eine DNA, die ein insektizides Cry1Be-Protein kodiert, und erzeugend ein vierten Protein ausgewählt aus der Gruppe bestehend aus Cry2A, Cry1I, Cry1Ab und DIG-3, wobei die Refugiumssamen weniger als 10 % aller Samen in der Mischung umfassen.

**9.** Pflanze nach einem der Ansprüche 1 und 5 bis 7, wobei die Pflanze ausgewählt ist aus der Gruppe bestehend aus Mais, Sojabohnen und Baumwolle.

**10.** Pflanzenzelle einer Pflanze nach einem der Ansprüche 1 und 5 bis 7, wobei die Pflanzenzelle die DNA, die das insektizide Cry1F-Protein kodiert, und die DNA, die das insektizide Vip3Ab-Protein kodiert, umfasst und exprimiert, wobei das insektizide Cry1F-Protein wenigstens 99 % identisch mit SEQ ID NO: 1 ist und das insektizide Vip3Ab-Protein wenigstens 99 % identisch mit SEQ ID NO: 2 ist.

**11.** Pflanze nach einem der Ansprüche 1 und 5 bis 7, wobei das insektizide Cry1F-Protein die SEQ ID NO: 1 umfasst und das insektizide Vip3Ab-Protein die SEQ ID NO: 2 umfasst.

**Revendications**

**1.** Plante transgénique comprenant et exprimant un ADN codant une protéine insecticide Vip3Ab et un ADN codant une protéine insecticide Cry1F, en outre comprenant et exprimant un ADN codant une troisième protéine insecticide, ladite troisième protéine étant choisie dans l'ensemble constitué par Cry1C, Cry1D et Cry1Be.

**2.** Semence d'une plante transgénique comprenant et exprimant l'ADN codant une protéine insecticide Vip3Ab et l'ADN codant une protéine Cry1F, en outre comprenant et exprimant un ADN codant une troisième protéine insecticide, ladite troisième protéine étant choisie dans l'ensemble constitué par Cry1C, Cry1D et Cry1Be.

**3.** Mélange de semences comprenant des semences refuges provenant de plantes refuges non-Bt, et une pluralité de semences selon la revendication 2, dans lequel lesdites semences refuges constituent moins de 40 % de la totalité des semences dans le mélange.

**4.** Composition destinée à la lutte contre des ravageurs lépidoptères, comprenant une cellule hôte transformée pour exprimer des quantités efficaces d'une protéine contenant la toxine de partie centrale Cry1F et une protéine Vip3Ab, comprenant encore une troisième protéine insecticide, la troisième protéine étant choisie dans l'ensemble constitué par Cry1C, Cry1D et Cry1Be, dans laquelle ladite protéine contenant la toxine de partie centrale Cry1F contient la partie toxine N-terminale, à activité insecticide, de Cry1F.

**5.** Plante transgénique selon la revendication 1, où ladite plante produit une quatrième protéine et une cinquième protéine choisie dans l'ensemble constitué par Cry2A, Cry1I, Cry1Ab et DIG-3.

**6.** Plante transgénique selon la revendication 1, où ladite plante produit une quatrième protéine choisie dans l'ensemble constitué par Cry2A, Cry1I, Cry1Ab et DIG-3.

**7.** Plante transgénique selon la revendication 6, dans laquelle ladite troisième protéine est une protéine CrylBe.

**8.** Mélange de semences comprenant des semences refuges provenant de plantes refuges non-Bt, et une pluralité de semences provenant d'une plante selon la revendication 7 comprenant et exprimant l'ADN codant une protéine insecticide Vip3Ab et l'ADN codant une protéine Cry1F, en outre comprenant et exprimant un ADN codant une protéine insecticide Cry1Be, et produisant une quatrième protéine choisie dans l'ensemble constitué par Cry2A, Cry1I, CrylAb et DIG-3, dans lequel lesdites semences refuges constituent moins de 10 % de la totalité des semences dans le mélange.

**9.** Plante selon l'une quelconque des revendications 1 et 5 à 7, où ladite plante est choisie dans l'ensemble constitué par le maïs, le soja et le cotonnier.

**10.** Cellule végétale d'une plante selon l'une quelconque des revendications 1 et 5 à 7, où ladite cellule végétale comprend et exprime ledit ADN codant ladite protéine insecticide Cry1F et ledit ADN codant ladite protéine insecticide Vip3Ab, ladite protéine insecticide Cry1F ayant un degré d'identité d'au moins 99 % avec la SEQ ID NO: 1, et ladite protéine insecticide Vip3Ab ayant un degré d'identité d'au moins 99 % avec la SEQ ID NO: 2.

**11.** Plante selon l'une quelconque des revendications 1 et 5 à 7, dans laquelle ladite protéine insecticide Cry1F comprend la SEQ ID NO: 1, et ladite protéine insecticide Vip3Ab comprend la SEQ ID NO: 2.

Figure 1. Growth inhibition (bars) and mortality (♦) dose responses for full length Vip3Ab1 against wild type *Spodoptera frugiperda* (J.E. Smith), (FAW) and Cry1Fa resistant type *Spodoptera frugiperda* (J.E. Smith), (rFAW). Percent growth inhibition is based upon comparison of average weight of 8 larvae treated with buffer only to the weight of larvae exposed to the toxin for 5 days.

Figure 2. Phosphor-image of $^{125}$I Cry1Fa bound to BBMV's from *S. frugiperda* after being separated by SDS-PAGE. Samples done in duplicate. Concentration of $^{125}$I Cry1Fa was 1 nM. Control represents level of binding of $^{125}$I Cry1Fa to BBMV's in the absence of any competitive ligand. 1,000 nM Cry1Fa represents the level of binding of $^{125}$I Cry1Fa to BBMV's in the presence of 1,000 nM non-radiolabeled Cry1Fa, showing complete displacement of the radiolabeled ligand from the BBMV protein. 1,000 nM Vip3Ab1 represents the level of binding of $^{125}$I Cry1Fa to BBMV's in the presence of 1,000 nM non-radiolabeled Vip3Ab1, showing that this protein does not have the ability to displace $^{125}$I Cry1Fa from *S. frugiperda* BBMV's even when added at 1,000-times the concentration of the radiolabeled ligand.

Figure 3. Phosphor-image of $^{125}$I Cry1Fa bound to BBMV's from wild type *S. frugiperda* (FAW) or Cry1Fa resistant *S. frugiperda* (rFAW), after being separated by SDS-PAGE. Samples done in duplicate. Concentration of $^{125}$I Cry1Fa was 2.5 nM. *FAW-0* represents level of binding of $^{125}$I Cry1Fa to wild type *S. frugiperda* BBMV's in the absence of any competitive ligand. *FAW-1,000 nM Cry1Fa* represents the level of binding of $^{125}$I Cry1Fa to wild type *S. frugiperda* BBMV's in the presence of 1,000 nM non-radiolabeled Cry1Fa, showing displacement of the radiolabeled ligand from the BBMV protein. *rFAW-0* represents level of binding of $^{125}$I Cry1Fa to Cry1Fa resistant *S. frugiperda* BBMV's in the absence of any competitive ligand. Note the absence of binding of $^{125}$I Cry1Fa to the BBMV's from resistant FAW. *rFAW-1,000 nM Cry1Fa* represents the level of binding of $^{125}$I Cry1Fa to BBMV's in the presence of 1,000 nM non-radiolabeled Vip3Ab1, again showing the inability of $^{125}$I Cry1Fa to bind to BBMV's from Cry1Fa resistant *S. frugiperda*.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090313717 A
- WO 2009132850 A
- US 20080311096 A
- US 61284281 B
- US 61284252 B
- US 61284290 B
- US 61284278 B
- US 201000269223 A
- US 5188960 A
- US 5827514 A
- US 6218188 B
- WO 9316094 A
- US 5135867 A
- US 4695455 A

- US 4965462 A
- EP 120516 A
- US 5380831 A
- US 6673990 B
- US 6551962 B
- US 6137033 A
- US 6656908 B
- WO 9818932 A2
- US 6603063 B
- WO 9957282 A2
- US 20040128716 A
- WO 03075655 A
- WO 02078437 A

### Non-patent literature cited in the description

- **MCGAUGHEY et al.** B.t. Resistance Management. *Nature Biotechnol.,* 1998, vol. 16, 144-146
- **TABASHNIK.** *PNAS,* 2008, vol. 105 (49), 19029-19030
- **N. CRICKMORE ; D.R. ZEIGLER ; J. FEITELSON ; E. SCHNEPF ; J. VAN RIE ; D. LERECLUS ; J. BAUM ; D.H. DEAN.** Revision of the Nomenclature for the Bacillus thuringiensis Pesticidal Crystal Proteins. *Microbiology and Molecular Biology Reviews,* 1998, vol. 62, 807-813
- **KELLER, G. H. ; M. M. MANAK.** DNA Probes. Stockton Press, 1987, 169-170
- **HUMASON ; GRETCHEN L.** Animal Tissue Techniques. W. H. Freeman and Company, 1967
- **POEHLMAN ; SLEPER.** Breeding Field Crops. 1995, 172-175
- **FEHR.** *Principles of Cultivar Development, Vol. 1: Theory and Technique,* 1987, vol. 1, 360-376
- for example, outlines two-toxin strategies, also called "pyramiding" or "stacking," for management of insecticidal transgenic crops. **ROUSH et al.** Phil. Trans. R. Soc. Lond. B. The Royal Society, 1998, vol. 353, 1777-1786
- **HECKEL,D.G. ; GAHAN,L.J. ; BAXTER,S.W. ; ZHAO,J.Z. ; SHELTON,A.M. ; GOULD,F. ; TABASHNIK,B.E.** The diversity of Bt resistance genes in species of Lepidoptera. *J Invertebr Pathol,* 2007, vol. 95, 192-197

- **LUO,K. ; BANKS,D. ; ADANG,M.J.** Toxicity, binding, and permeability analyses of four bacillus thuringiensis cry1 delta-endotoxins using brush border membrane vesicles of spodoptera exigua and spodoptera frugiperda. *Appl. Environ. Microbiol.,* 1999, vol. 65, 457-464
- **PALMER, M. ; BUCHKREMER, M ; VALEVA, A ; BHAKDI, S.** Cysteine-specific radioiodination of proteins with fluorescein maleimide. *Analytical Biochemistry,* 1997, vol. 253, 175-179
- **SAMBROOK,J. ; RUSSELL,D.W.** Molecular Cloning: A Laboratory Manual. Cold Spring Harbor Laboratory, 2001
- **SCHLENZ, M. L. ; BABCOCK, J. M. ; STORER, N. P.** Response of Cry1F-resistant and Susceptible European Corn Borer and Fall Armyworm Colonies to Cry1A.105 and Cry12Ab2. *Indianapolis, Dow Agro-Sciences,* 2008
- **SHEETS, J. J. ; STORER, N. P.** Analysis of Cry1Ac Binding to Proteins in Brush Border Membrane Vesicles of Corn Earworm Larvae (Heleothis zea). *Interactions with Cry1F Proteins and Its Implication for Resistance in the Field. DAI-0417,* 2001, 1-26
- **TABASHNIK,B.E. ; LIU,Y.B. ; FINSON,N. ; MASSON,L. ; HECKEL,D.G.** One gene in diamondback moth confers resistance to four Bacillus thuringiensis toxins. *Proc. Natl. Acad. Sci. U. S. A,* 1997, vol. 94, 1640-1644

- **TABASHNIK,B.E. ; MALVAR,T. ; LIU,Y.B. ; FINSON,N. ; BORTHAKUR,D. ; SHIN,B.S. ; PARK,S.H. ; MASSON,L. ; DE MAAGD,R.A. ; BOSCH,D.** Cross-resistance of the diamondback moth indicates altered interactions with domain II of Bacillus thuringiensis toxins. *Appl. Environ. Microbiol.,* 1996, vol. 62, 2839-2844
- **TABASHNIK,B.E. ; ROUSH,R.T. ; EARLE,E.D. ; SHELTON,A.M.** *Resistance to Bt toxins. Science,* 2000, vol. 287, 42
- **WOLFERSBERGER,M.G.** Preparation and partial characterization of amino acid transporting brush border membrane vesicles from the larval midgut of the gypsy moth (Lymantria dispar). *Arch. Insect Biochem. Physiol,* 1993, vol. 24, 139-147
- **XU,X. ; YU,L. ; WU,Y.** Disruption of a cadherin gene associated with resistance to Cry1Ac {delta}-endotoxin of Bacillus thuringiensis in Helicoverpa armigera. *Appl Environ Microbiol,* 2005, vol. 71, 948-954
- **ESTRUCH.** *PNAS,* 1996, vol. 93, 5389-5394
- **DOSS.** *Protein Expr Purif,* 2002, vol. 26, 82-88
- **CHEN.** *Sheng Wu Gong Cheng Xue Bao,* 2002, vol. 18, 687-692
- **MESRATI.** *FEMS Micro Lett,* 2005, vol. 244, 353-358

SEQUENCE LISTING

<110> Dow AGROSCIENCES LLC

<120> COMBINED USE OF CRY1FA AND VIP3AB1 FOR MANAGEMENT OF RESISTANT INSECTS

<130> DAS-P0177-US

<160> 2

<170> PatentIn version 3.5

<210> 1
<211> 605
<212> PRT
<213> Artificial Sequence

<220>
<223> Cry1Fa

<400> 1

```
Met Glu Asn Asn Ile Gln Asn Gln Cys Val Pro Tyr Asn Cys Leu Asn
1               5               10              15

Asn Pro Glu Val Glu Ile Leu Asn Glu Glu Arg Ser Thr Gly Arg Leu
            20              25              30

Pro Leu Asp Ile Ser Leu Ser Leu Thr Arg Phe Leu Leu Ser Glu Phe
            35              40              45

Val Pro Gly Val Gly Val Ala Phe Gly Leu Phe Asp Leu Ile Trp Gly
    50              55              60

Phe Ile Thr Pro Ser Asp Trp Ser Leu Phe Leu Leu Gln Ile Glu Gln
65              70              75              80

Leu Ile Glu Gln Arg Ile Glu Thr Leu Glu Arg Asn Arg Ala Ile Thr
            85              90              95

Thr Leu Arg Gly Leu Ala Asp Ser Tyr Glu Ile Tyr Ile Glu Ala Leu
            100             105             110

Arg Glu Trp Glu Ala Asn Pro Asn Asn Ala Gln Leu Arg Glu Asp Val
            115             120             125

Arg Ile Arg Phe Ala Asn Thr Asp Asp Ala Leu Ile Thr Ala Ile Asn
    130             135             140

Asn Phe Thr Leu Thr Ser Phe Glu Ile Pro Leu Leu Ser Val Tyr Val
145             150             155             160

Gln Ala Ala Asn Leu His Leu Ser Leu Leu Arg Asp Ala Val Ser Phe
            165             170             175
```

```
Gly Gln Gly Trp Gly Leu Asp Ile Ala Thr Val Asn Asn His Tyr Asn
            180                 185                 190

Arg Leu Ile Asn Leu Ile His Arg Tyr Thr Lys His Cys Leu Asp Thr
            195                 200                 205

Tyr Asn Gln Gly Leu Glu Asn Leu Arg Gly Thr Asn Thr Arg Gln Trp
    210                 215                 220

Ala Arg Phe Asn Gln Phe Arg Arg Asp Leu Thr Leu Thr Val Leu Asp
225                 230                 235                 240

Ile Val Ala Leu Phe Pro Asn Tyr Asp Val Arg Thr Tyr Pro Ile Gln
                245                 250                 255

Thr Ser Ser Gln Leu Thr Arg Glu Ile Tyr Thr Ser Ser Val Ile Glu
            260                 265                 270

Asp Ser Pro Val Ser Ala Asn Ile Pro Asn Gly Phe Asn Arg Ala Glu
            275                 280                 285

Phe Gly Val Arg Pro Pro His Leu Met Asp Phe Met Asn Ser Leu Phe
    290                 295                 300

Val Thr Ala Glu Thr Val Arg Ser Gln Thr Val Trp Gly Gly His Leu
305                 310                 315                 320

Val Ser Ser Arg Asn Thr Ala Gly Asn Arg Ile Asn Phe Pro Ser Tyr
            325                 330                 335

Gly Val Phe Asn Pro Gly Gly Ala Ile Trp Ile Ala Asp Glu Asp Pro
            340                 345                 350

Arg Pro Phe Tyr Arg Thr Leu Ser Asp Pro Val Phe Val Arg Gly Gly
            355                 360                 365

Phe Gly Asn Pro His Tyr Val Leu Gly Leu Arg Gly Val Ala Phe Gln
    370                 375                 380

Gln Thr Gly Thr Asn His Thr Arg Thr Phe Arg Asn Ser Gly Thr Ile
385                 390                 395                 400

Asp Ser Leu Asp Glu Ile Pro Pro Gln Asp Asn Ser Gly Ala Pro Trp
            405                 410                 415

Asn Asp Tyr Ser His Val Leu Asn His Val Thr Phe Val Arg Trp Pro
            420                 425                 430
```

```
Gly Glu Ile Ser Gly Ser Asp Ser Trp Arg Ala Pro Met Phe Ser Trp
        435             440         445

Thr His Arg Ser Ala Thr Pro Thr Asn Thr Ile Asp Pro Glu Arg Ile
        450             455         460

Thr Gln Ile Pro Leu Val Lys Ala His Thr Leu Gln Ser Gly Thr Thr
465             470             475             480

Val Val Arg Gly Pro Gly Phe Thr Gly Gly Asp Ile Leu Arg Arg Thr
            485             490             495

Ser Gly Gly Pro Phe Ala Tyr Thr Ile Val Asn Ile Asn Gly Gln Leu
        500             505             510

Pro Gln Arg Tyr Arg Ala Arg Ile Arg Tyr Ala Ser Thr Thr Asn Leu
        515             520             525

Arg Ile Tyr Val Thr Val Ala Gly Glu Arg Ile Phe Ala Gly Gln Phe
        530             535             540

Asn Lys Thr Met Asp Thr Gly Asp Pro Leu Thr Phe Gln Ser Phe Ser
545             550             555             560

Tyr Ala Thr Ile Asn Thr Ala Phe Thr Phe Pro Met Ser Gln Ser Ser
            565             570             575

Phe Thr Val Gly Ala Asp Thr Phe Ser Ser Gly Asn Glu Val Tyr Ile
            580             585             590

Asp Arg Phe Glu Leu Ile Pro Val Thr Ala Thr Leu Glu
            595             600             605
```

<210> 2
<211> 788
<212> PRT
<213> Artificial Sequence

<220>
<223> Vip3Ab1

<400> 2

```
Met Ala Asn Met Asn Asn Thr Lys Leu Asn Ala Arg Ala Leu Pro Ser
1               5                   10                  15
```

```
Phe Ile Asp Tyr Phe Asn Gly Ile Tyr Gly Phe Ala Thr Gly Ile Lys
            20                  25                  30
```

```
Asp Ile Met Asn Met Ile Phe Lys Thr Asp Thr Gly Gly Asn Leu Thr
            35                  40                  45
```

```
Leu Asp Glu Ile Leu Lys Asn Gln Gln Leu Leu Asn Glu Ile Ser Gly
    50              55              60

Lys Leu Asp Gly Val Asn Gly Ser Leu Asn Asp Leu Ile Ala Gln Gly
65              70              75              80

Asn Leu Asn Thr Glu Leu Ser Lys Glu Ile Leu Lys Ile Ala Asn Glu
            85              90              95

Gln Asn Gln Val Leu Asn Asp Val Asn Asn Lys Leu Asp Ala Ile Asn
            100             105             110

Thr Met Leu His Ile Tyr Leu Pro Lys Ile Thr Ser Met Leu Ser Asp
        115             120             125

Val Met Lys Gln Asn Tyr Ala Leu Ser Leu Gln Val Glu Tyr Leu Ser
        130             135             140

Lys Gln Leu Lys Glu Ile Ser Asp Lys Leu Asp Val Ile Asn Val Asn
145             150             155             160

Val Leu Ile Asn Ser Thr Leu Thr Glu Ile Thr Pro Ala Tyr Gln Arg
            165             170             175

Ile Lys Tyr Val Asn Glu Lys Phe Glu Glu Leu Thr Phe Ala Thr Glu
        180             185             190

Thr Thr Leu Lys Val Lys Lys Asp Ser Ser Pro Ala Asp Ile Leu Asp
        195             200             205

Glu Leu Thr Glu Leu Thr Glu Leu Ala Lys Ser Val Thr Lys Asn Asp
    210             215             220

Val Asp Gly Phe Glu Phe Tyr Leu Asn Thr Phe His Asp Val Met Val
225             230             235             240

Gly Asn Asn Leu Phe Gly Arg Ser Ala Leu Lys Thr Ala Ser Glu Leu
            245             250             255

Ile Ala Lys Glu Asn Val Lys Thr Ser Gly Ser Glu Val Gly Asn Val
        260             265             270

Tyr Asn Phe Leu Ile Val Leu Thr Ala Leu Gln Ala Lys Ala Phe Leu
        275             280             285

Thr Leu Thr Thr Cys Arg Lys Leu Leu Gly Leu Ala Asp Ile Asp Tyr
    290             295             300
```

Thr Ser Ile Met Asn Glu His Leu Asn Lys Glu Lys Glu Glu Phe Arg
305                     310                 315                 320

Val Asn Ile Leu Pro Thr Leu Ser Asn Thr Phe Ser Asn Pro Asn Tyr
                325                 330                 335

Ala Lys Val Lys Gly Ser Asp Glu Asp Ala Lys Met Ile Val Glu Ala
                340                 345                 350

Lys Pro Gly His Ala Leu Val Gly Phe Glu Ile Ser Asn Asp Ser Met
                355                 360                 365

Thr Val Leu Lys Val Tyr Glu Ala Lys Leu Lys Gln Asn Tyr Gln Val
    370                 375                 380

Asp Lys Asp Ser Leu Ser Glu Val Ile Tyr Ser Asp Met Asp Lys Leu
385                 390                 395                 400

Leu Cys Pro Asp Gln Ser Glu Gln Ile Tyr Tyr Thr Asn Asn Ile Val
                405                 410                 415

Phe Pro Asn Glu Tyr Val Ile Thr Lys Ile Asp Phe Thr Lys Lys Met
                420                 425                 430

Lys Thr Leu Arg Tyr Glu Val Thr Ala Asn Ser Tyr Asp Ser Ser Thr
                435                 440                 445

Gly Glu Ile Asp Leu Asn Lys Lys Lys Val Glu Ser Ser Glu Ala Glu
    450                 455                 460

Tyr Arg Thr Leu Ser Ala Asn Asn Asp Gly Val Tyr Met Pro Leu Gly
465                 470                 475                 480

Val Ile Ser Glu Thr Phe Leu Thr Pro Ile Asn Gly Phe Gly Leu Gln
                485                 490                 495

Ala Asp Glu Asn Ser Arg Leu Ile Thr Leu Thr Cys Lys Ser Tyr Leu
                500                 505                 510

Arg Glu Leu Leu Leu Ala Thr Asp Leu Ser Asn Lys Glu Thr Lys Leu
                515                 520                 525

Ile Val Pro Pro Ile Ser Phe Ile Ser Asn Ile Val Glu Asn Gly Asn
    530                 535                 540

Leu Glu Gly Glu Asn Leu Glu Pro Trp Ile Ala Asn Asn Lys Asn Ala
545                 550                 555                 560

Tyr Val Asp His Thr Gly Gly Ile Asn Gly Thr Lys Val Leu Tyr Val

```
                    565                      570                      575

His Lys Asp Gly Glu Phe Ser Gln Phe Val Gly Gly Lys Leu Lys Ser
            580                  585                  590

Lys Thr Glu Tyr Val Ile Gln Tyr Ile Val Lys Gly Lys Ala Ser Ile
            595                  600                  605

Tyr Leu Lys Asp Lys Lys Asn Glu Asn Ser Ile Tyr Glu Glu Ile Asn
    610                  615                  620

Asn Asp Leu Glu Gly Phe Gln Thr Val Thr Lys Arg Phe Ile Thr Gly
625                  630                  635                  640

Thr Asp Ser Ser Gly Ile His Leu Ile Phe Thr Ser Gln Asn Gly Glu
            645                  650                  655

Gly Ala Phe Gly Gly Asn Phe Ile Ile Ser Glu Ile Arg Thr Ser Glu
            660                  665                  670

Glu Leu Leu Ser Pro Glu Leu Ile Met Ser Asp Ala Trp Val Gly Ser
            675                  680                  685

Gln Gly Thr Trp Ile Ser Gly Asn Ser Leu Thr Ile Asn Ser Asn Val
    690                  695                  700

Asn Gly Thr Phe Arg Gln Asn Leu Pro Leu Glu Ser Tyr Ser Thr Tyr
705                  710                  715                  720

Ser Met Asn Phe Thr Val Asn Gly Phe Gly Lys Val Thr Val Arg Asn
            725                  730                  735

Ser Arg Glu Val Leu Phe Glu Lys Ser Tyr Pro Gln Leu Ser Pro Lys
            740                  745                  750

Asp Ile Ser Glu Lys Phe Thr Thr Ala Ala Asn Asn Thr Gly Leu Tyr
            755                  760                  765

Val Glu Leu Ser Arg Ser Thr Ser Gly Gly Ala Ile Asn Phe Arg Asp
    770                  775                  780

Phe Ser Ile Lys
785
```